# EUROPÄISCHE PATENTSCHRIFT

(11) **EP 1 080 089 B1**
(45) Veröffentlichungstag und Bekanntmachung des Hinweises auf die Patenterteilung: **05.11.2003**
(21) Anmeldenummer: 99924925.3
(22) Anmeldetag: 10.05.1999
(51) Int. Cl.: C07D 409/06, C07D 409/14, A01N 43/56

(54) **PYRAZOLYLDIOXOTHIOCHROMANOYL-DERIVATE**
PYRAZOLYLDIOXOTHIOCHROMANOYL DERIVATIVES
DERIVES DU TYPE PYRAZOLYLDIOXOTHIOCHROMANOYLE

(30) Priorität: 18.05.1998 DE 19822213
(43) Veröffentlichungstag der Anmeldung: 07.03.2001
(73) Patentinhaber: BASF AKTIENGESELLSCHAFT, 67056 Ludwigshafen (DE)
(72) Erfinder: WITSCHEL, Matthias, D-67061 Ludwigshafen (DE); LANGEMANN, Klaus, D-67551 Worms (DE); VON DEYN, Wolfgang, D-67435 Neustadt (DE); MISSLITZ, Ulf, D-67433 Neustadt (DE); BAUMANN, Ernst, D-67373 Dudenhofen (DE); ENGEL, Stefan, D-55268 Nieder-Olm (DE); MAYER, Guido, D-67433 Neustadt (DE); NEIDLEIN, Ulf, D-68165 Mannheim (DE); WAGNER, Oliver, D-67433 Neustadt (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: EP9903197
(87) Internationale Veröffentlichungsnummer: WO99059991

(56) Entgegenhaltungen:
- EP-A- 0 629 623
- EP-A- 0 712 853
- EP-A- 0 728 756
- WO-A-97/30993
- DE-A- 19 532 312

## Beschreibung

Die vorliegende Erfindung betrifft neue Pyrazolyldioxothiochromanoyl-Derivate der Formel I, 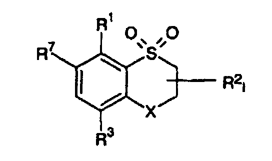 in der die Variablen folgende Bedeutungen haben:
- X: S(=O)₂ oder, CR⁴R⁵ ;
- R¹: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino. N-(C₁-C₆-Alkyl)-N-(C₁-C₆-alkylsulfonyl)-amino oder N-(C₁-C₆-Alky)-N-(C₁-C₆-halogenalkylsulfonyl)-amino;
- R²: C₁-C₆-Alkyl, C₁-C₆-Halogenalkyl, C₁-C₆-Alkoxy oder C₁-C₆-Halogenalkoxy;
- R³: Wasserstoff, C₁-C₆-Alkyl oder Halogen;
- R⁴,R⁵: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl. C₁-C₆-Alkylsulfonyl, C₁-C₆-Halogenalkylsulfonyl, N-C₁-C₆-Alkylamino, N-C₁-C₆-Halogenalkylamino, N,N-Di-(C₁-C₆-alkyl)-amino, N-C₁-C₆-Alkoxyamino, N-(C₁-C₆-Alkoxy)-N-(C₁-C₆-alkyl)amino, 1-Tetrahydropyrrolyl, 1-Piperidinyl, 4-Morpholinyl oder 1-Hexahydropyrazinyl;
oder
- R⁴ und R⁵: bilden gemeinsam eine -O-(CH₂)ₘ-O-, -O-(CH₂)ₘ-S-, -S-(CH₂)ₘ-S- oder -O-(CH₂)ₙ-Kette, die durch einen bis drei Reste aus folgender Gruppe substituiert sein kann:
Halogen, Cyano, C₁-C₄-Alkyl, C₁-C₄-Halogenalkyl oder C₁-C₄-Alkoxycarbonyl;
oder
- R⁴ und R⁵: bilden gemeinsam eine -(CH₂)ₚ-Kette, die durch Sauerstoff oder Schwefel unterbrochen sein kann und/oder durch einen bis vier Reste aus folgender Gruppe substituiert sein kann:
Halogen, Cyano, C₁-C₄-Alkyl, C₁-C₄-Halogenalkyl oder C₁-C₄-Alkoxycarbonyl;
oder
- R⁴ und R⁵: bilden gemeinsam eine Methylidengruppe, die durch einen bis zwei Reste aus folgender Gruppe substituiert sein kann:
Halogen, Cyano, Hydroxy, Formyl, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- l: 0 bis 4;
- m: 2 bis 4;
- n: 1 bis 5;
- p: 2 bis 5;
- R⁷: eine Verbindung IIa 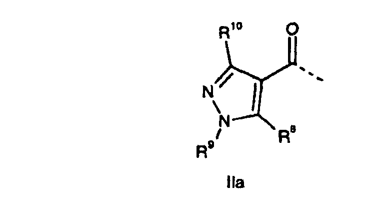
wobei
- R⁸: Halogen, OR¹¹, SR¹¹, SOR¹², SO₂R¹², POR¹²R¹³, OPOR¹²R¹³, OPSR¹²R¹³, NR¹⁴R¹⁵, ONR¹⁵R¹⁵, N-gebundenes Heterocyclyl oder O-(N-gebundenes Heterocyclyl), wobei der Heterocyclyl-Rest der beiden letztge- nannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R⁹: Wasserstoff, C₁-C₆-Alkyl, C₁-C₆-Halogenalkyl, Hydroxy, C₁-C₆-Alkoxy oder C₁-C₆-Halogenalkoxy;
- R¹⁰: Wasserstoff, Halogen, C₁-C₆-Alkyl, C₁-C₆-Halogenalkyl, Hydroxy, C₁-C₆-Alkoxy, C₁-C₆-Halogenalkoxy, C₁-C₆-Alkylthio oder C₁-C₆-Halogenalkylthio;
- R¹¹: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₂₀-Alkylcarbonyl, C₂-C₂₀-Alkenylcarbonyl, C₂-C₆-Alkinylcarbonyl, C₃-C₆-Cycloalkylcarbonyl, (2-Norbornyl)methylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Phenyloxythiocarbonyl, Heterocyclyloxycarbonyl, Heterocyclyloxythiocarbonyl, Phenylaminocarbonyl, N-(C₁-C₆-Alkyl)-N-(phenyl)-aminocarbonyl, Heterocyclylaminocarbonyl. N-(C₁-C₆-Alkyl)-N-(heterocyclyl)-aminocarbonyl, Phenyl-C₂-C₆-alkenylcarbonyl oder Heterocyclyl-C₂-C₆-alkenylcarbonyl, wobei der Phenyl- und der Heterocyclyl-Rest der 18 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Phenoxy, Heterocyclyl oder N-gebundenes Heterocyclyl, wobei die drei letztgenannten Substituenten ihrerseits partiell oder vollständig halogeniert sein können und/oder einen bis drei der folgenden Reste tragen können: Nitro, Cyano, C₁-C₄-Alkyl, C₁-C₄-Halogenalkyl, C₁-C₄-Alkoxy oder C₁-C₄-Halogenalkoxy;
- R¹², R¹³: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, Amino, C₁-C₆-Alkylamino, C₁-C₆-Halogenalkylamino, Di-(C₁-C₆-alkyl)amino oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di- (C₁-C₄-alkyl) -aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁴: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-Alkyl)-amino oder C₁-C₆-Alkylcarbonylamino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁵: C₁-C₆-Alkyl, C₃-C₆-Alkenyl, C₃-C₆-Alkinyl oder C₁-C₆-Alkylcarbonyl;
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus DE-A 19 532 312 , WO 97/30993 und WO 97/08164 sind Dioxothiochroman-Derivate, die mit einem substituierten (5-Hydroxypyrazol-4-yl)carbonyl-Rest verknüpft sind, bekannt. Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, biologisch, insbesondere herbizid wirksame, Verbindungen mit verbesserten Eigenschaften zu finden.

Demgemäß wurden die Pyrazolyldioxothiochromanoyl-Derivate der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹⁵ oder als Reste an Phenyl- und Heterocyclyl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl-, N,N-Dialkylaminosulfonyl-, N-Alkylamino-, N,N-Dialkylamino-, N-Halogenalkylamino-, N,N-Dihalogenalkylamino, N-Alkoxyamino-, N-Alkoxy-N-alkylamino-, N-Alkylcarbonylamino-, N-Alkylsulfonylamino-, N-Halogenalkylsulfonylamino-, N-Alkyl-N-alkylsulfonylamino-, N-Alkyl-N-halogenalkylsulfonylamino-, Alkylcarbonyl-, Alkoxycarbonyl-, Alkylthiocarbonyl-, Alkylcarbonyloxy-, Alkylaminocarbonyl-, Dialkylaminocarbonyl-, Dialkylaminothiocarbonyl-, Alkoxyalkyl-, Alkylcarbonylalkyl-, Alkoxyiminoalkyl-, N-(Alkylamino)-iminoalkyl-, N-(Dialkylamino)-iminoalkyl-, Phenylalkenylcarbonyl-, Heterocyclylalkenylcarbonyl-, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Dialkylaminoalkoxycarbonyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl-, Alkenyloxy- und Alkinyloxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: z.B. Methyl, Ethyl, Propyl, 1-Methylethyl, Butyl, 1-Methylpropyl, 2-Methylpropyl oder 1,1-Dimethylethyl;
- C₁-C₆-Alkyl, sowie die Alkylteile von C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl, N(C₁-C₆-Alkoxy)-N-(C₁-C₆-alkyl)-aminocarbonyl, N-(C₃-C₆-Alkenyl-N-(C₁-C₆-alkyl)-aminocarbonyl, (C₃-C₆-Alkinyl)-N-(C₁-C₆-alkyl)-aminocarbonyl, N-(C₁-C₆-Alkyl)-N-phenylaminocarbonyl, N-(C₁-C₆-Alkyl)-N-heterocyclylaminocarbonyl, Phenyl-C₁-C₆-alkyl, N-(C₁-C₆-Alkyl)-N-(C₁-C₆-alkylsulfonyl)-amino, N-(C₁-C₆-Alkyl)-N-(C₁-C₆-halogenalkylsulfonyl)-amino, Heterocyclyl-C₁-C₆-alkyl, Phenylcarbonyl-C₁-C₆-alkyl, Heterocyclylcarbonyl-C₁-C₆-alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 2,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl. 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl, sowie die Halogenalkylteile von N-C₁-C₆-Halogenalkylamino und N,N-(Di-C₁-C₆-halogenalkyl)amino:
   C₁-C₄-Halogenalkyl, wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy: z.B. Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von N-C₁-C₆-Alkoxyamino. N-C₁-C₆-Alkoxy-N-C₁-C₆ - alkylamino, C₁-C₆-Alkoxyimino-C₁-C₆-alkyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl und N-(C₃-C₆-Alkinyl)-N-(C₁-C₆-alkoxy)-aminocarbonyl: C₁-C₄-Alkoxy, wie voranstehend genannt, sowie z.B. Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio : z.B. Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio, sowie die Alkylthioteile von C₁-C₆-Alkylthiocarbonyl; C₁-C₄-Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1, 2-Dimethylbutylthio. 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₆-Halogenalkylthio: einen C₁-C₄-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio, Nonafluorbutylthio, 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl. 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl) -2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₆-Alkylsulfonyl (C₁-C₆-Alkyl-S(=O)₂-), sowie die Alkylsulfonylreste von N-(C₁-C₆-Alkylsulfonyl)-amino und N-(C₁-C₆-Alkyl)-N-(C₁-C₆-alkylsulfonyl)-amino: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methyipropyisulfonyl;
- C₁-C₆-Halogenalkylsulfonyl, sowie die Halogenalkylsulfonylreste von N-(C₁-C₆-Haloqenalkylsulfonyl)-amino und N-(C₁-C₆-Alkyl)-N-(C₁-C₆-halogenalkylsulfonyl)-amino : einen C₁-C₆-Alkylsulfonylrest, wie voranstehend genannt, der partiell oder vollständig durch Fluor, Chlor, Brom und/oder Iod substituiert ist, also z.B. Fluonnethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichiorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₁-C₆-Alkylamino, sowie die Alkylaminoreste von N-(C₁-C₆-Alkoxy)-N-(C₁-C₆-alkyl)amino und N-(C₁-C₆-Alkylamino)-imino-C₁-C₆-alkyl, also z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- (C₁-C₄-Alkylamino)sulfonyl: z.B. Methylaminosulfonyl, Ethylaminosuifonyl, Propylaminosulfonyl, 1-Methylethylaminosulfonyl, Butylaminosulfonyl, 1-Methylpropylaminosulfonyl, 2-Methylpropylaminosulfonyl oder 1,1-Dimethylethylaminosulfonyl;
- (C₁-C₆-Alkylamino)sulfonyl: (C₁-C₄-Alkylamino)sulfonyl, wie vorstehend genannt, sowie z.B. Pentylaminosulfonyl, 1-Methylbutylaminosulfonyl, 2-Methylbutylaminosulfonyl, 3-Methylbutylaminosulfonyl, 2,2-Dimethylpropylaminosulfonyl, 1-Ethylpropylaminosulfonyl, Hexylaminosulfonyl, 1,1-Dimethylpropylaminosulfonyl, 1,2-Dimethylpropylaminosulfonyl, 1-Methylpentylaminosulfonyl, 2-Methylpentylaminosulfonyl, 3-Methylpentylaminosulfonyl, 4-Methylpentylaminosulfonyl, 1,1-Dimethylbutylaminosulfonyl, 1,2-Dimethylbutylaminosulfonyl, 1,3-Dimethylbutylaminosulfonyl, 2,2-Dimethylbutylaminosulfonyl, 2,3-Dimethylbutylaminosulfonyl, 3,3-Dimethylbutylaminosulfonyl, 1-Ethylbutylaminosulfonyl, 2-Ethylbutylaminosulfonyl, 1,1,2-Trimethylpropylaminosulfonyl, 1,2,2-Trimethylpropylaminosulfonyl, 1-Ethyl-1-methylpropylaminosulfonyl oder 1-Ethyl-2-methylpropylaminosulfonyl;
- Di-(C₁-C₄-alkyl)-aminosulfonyl: z.B. N,N-Dimethylaminosulfonyl, N,N-Diethylaminosulfonyl, N,N-Di-(1-methylethyl) aminosulfonyl, N,N-Dipropylaminosulfonyl, N,N-Dibutylaminosulfonyl, N,N-Di-(1-methylpropyl)-aminosulfonyl, N,N-Di-(2-methylpropyl)-aminosulfonyl, N,N-Di-(1,1-dimethylethyl)-aminosulfonyl, N-Ethyl-N-methylaminosulfonyl, N-Methyl-N-propylaminosulfonyl, N-Methyl-N-(1-methylethyl)-aminosulfonyl)-N-Butyl-N-methylaminosulfonyl, N-Methyl-N-(1-methylpropyl)-aminosulfonyl, N-Methyl-N-(2-methylpropyl)-aminosulfonyl, N-(1,1-Dimethylethyl)-N-methylaminosulfonyl, N-Ethyl-N-propylaminosulfonyl, N-Ethyl-N-(1-methylethyl)-aminosulfonyl, N-Butyl-N-ethylaminosulfonyl, N-Ethyl-N-(1-methylpropyl)-aminosulfonyl, N-Ethyl-N-(2-methylpropyl)-aminosulfonyl, N-Ethyl-N-(1,1-dimethylethyl)-aminosulfonyl, N-(1-Methylethyl)-N-propylaminosulfonyl, N-Butyl-N-propylaminosulfonyl, N-(1-Methylpropyl)-N-propylaminosulfonyl, N-(2-Methylpropyl)-N-propylaminosulfonyl, N-(1,1-Dimethylethyl)-N-propylaminosulfonyl, N-Butyl-N-(1-methylethyl)-aminosulfonyl, N-(1-Methylethyl)-N-(1-methylpropyl)-aminosulfonyl, N-(1-Methylethyl)-N-(2-methylpropyl)-aminosulfonyl, N-(1,1-Dimethylethyl)-N-(1-methylethyl)-aminosulfonyl, N-Butyl-N-(1-methylpropyl)-aminosulfonyl, N-Butyl-N-(2-methylpropyl)-aminosulfonyl, N-Butyl-N-(1,1-dimethylethyl)-aminosulfonyl, N-(1-Methylpropyl)-N-(2-methylpropyl)-aminosulfonyl, N-(1,1-Dimethylethyl)-N-(1-methylpropyl)-aminosulfonyl oder N-(1,1-Dimethylethyl)-N-(2-methylpropyl)-aminosulfonyl;
- Di-(C₁-C₆-alkyl)-aminosulfonyl: Di-(C₁-C₄-alkyl)-aminosulfonyl, wie voranstehend genannt, sowie z.B. N-Methyl-N-pentylaminosulfonyl, N-Methyl-N-(1-methylbutyl)-aminosulfonyl, N-Methyl-N-(2-methylbutyl)-aminosulfonyl, N-Methyl-N-(3-methylbutyl)-aminosulfonyl, N-Methyl-N-(2,2-dimethylpropyl)-aminosulfonyl, N-Methyl-N-(1-ethylpropyl)-aminosulfonyl, N-Methyl-N-hexylaminosulfonyl, N-Methyl-N-(1,1-dimethylpropyl)-aminosulfonyl, N-Methyl-N-(1,2-dimethylpropyl)-aminosulfonyl, N-Methyl-N-(1-methylpentyl)-aminosulfonyl, N-Methyl-N-(2-methylpentyl)-aminosulfonyl, N-Methyl-N-(3-methylpentyl)-aminosulfonyl, N-Methyl-N-(4-methylpentyl)-aminosulfonyl, N-Methyl-N-(1,1-dimethylbutyl)-aminosulfonyl, N-Methyl-N-(1,2-dimethylbutyl)-aminosulfonyl, N-Methyl-N-(1,3-dimethylbutyl)-aminosulfonyl, N-Methyl-N-(2, 2-dimethylbutyl)-aminosulfonyl, N-Methyl-N-(2, 3-dimethylbutyl)-aminosulfonyl, N-Methyl-N-(3,3-dimethylbutyl)-aminosulfonyl, N-Methyl-N-(1-ethylbutyl)-aminosulfonyl, N-Methyl-N-(2-ethylbutyl)-aminosulfonyl, N-Methyl-N-(1,1,2-trimethylpropyl)-aminosulfonyl, N-Methyl-N-(1,2,2-trimethylpropyl)-aminosulfonyl, N-Methyl-N-(1-ethyl-1-methylpropyl)-aminosulfonyl, N-Methyl-N-(1-ethyl-2-methylpropyl)-aminosulfonyl, N-Ethyl-N-pentylaminosulfonyl, N-Ethyl-N-(1-methylbutyl)-aminosulfonyl, N-Ethyl-N-(2-methylbutyl)-aminosulfonyl, N-Ethyl-N-(3-methylbutyl)-aminosulfonyl, N-Ethyl-N-(2,2-dimethylpropyl)-aminosulfonyl, N-Ethyl-N-(1-ethylpropyl)-aminosulfonyl, N-Ethyl-N-hexylaminosulfonyl, N-Ethyl-N-(1,1-dimethylpropyl)-aminosulfonyl, N-Ethyl-N-(1,2-dimethylpropyl)-aminosulfonyl, N-Ethyl-N-(1-methylpentyl)-aminosulfonyl, N-Ethyl-N-(2-methylpentyl)-aminosulfonyl, N-Ethyl-N-(3-methylpentyl)-aminosulfonyl, N-Ethyl-N-(4-methylpentyl)-aminosulfonyl, N-Ethyl-N-(1,1-dimethylbutyl)-aminosulfonyl, N-Ethyl-N-(1,2-dimethylbutyl)-aminosulfonyl, N-Ethyl-N-(1,3-dimethylbutyl)-aminosulfonyl, N-Ethyl-N-(2,2-dimethylbutyl)-aminosulfonyl, N-Ethyl-N-(2,3-dimethylbutyl)-aminosulfonyl, N-Ethyl-N-(3,3-dimethylbutyl)-aminosulfonyl, N-Ethyl-N-(1-ethylbutyl)-aminosulfonyl, N-Ethyl-N-(2-ethylbutyl)-aminosulfonyl, N-Ethyl-N-(1,1,2-trimethylpropyl)-aminosulfonyl, N-Ethyl-N-(1,2,2-trimethylpropyl)-aminosulfonyl, N-Ethyl-N-(1-ethyl-1-methylpropyl)-aminosulfonyl, N-Ethyl-N-(1-ethyl-2-methylpropyl)-aminosulfonyl, N-Propyl-N-pentylaminosulfonyl, N-Butyl-N-pentylaminosulfonyl, N,N-Dipentylaminosulfonyl, N-Propyl-N-hexylaminosulfonyl, N-Butyl-N-hexylaminosulfonyl, N-Pentyl-N-hexylaminosulfonyl oder N,N-Dihexylaminosulfonyl;
- Di-(C₁-C₄-alkyl)amino, sowie die Dialkylaminoreste von Di-(C₁-C₄-alkyl)amino-C₁-C₄-alkoxycarbonyl und N-(Di-C₁-C₄alkylamino)-imino-C₁-C₆-alkyl, also z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl) amino, N-(1,1-Dimethylethyl)-N-methylaminol N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N- (2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
- Di-(C₁-C₆-alkyl)amino, sowie die Dialkylaminoreste von Di-(C₁-C₆-alkyl)amino-imino-C₁-C₆-alkyl: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylamino.
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonylamino, C₁-C₆-Alkylcarbonyl-C₁-C₆-alkyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1, 2-Dimethylbutylcarbonyl, 1,3-Dimethylbutylcarbonyl, 2,2.-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₁-C₂₀-Alkylcarbonyl: C₁-C₆-Alkylcarbonyl, wie voranstehend genannt, sowie Heptylcarbonyl, Octylcarbonyl, Pentadecylcarbonyl oder Heptadecylcarbonyl;
- C₁-C₄-Alkoxycarbonyl, sowie die Alkoxycarbonylteile von Di-(C₁-C₄-alkyl)amino-C₁-C₄-alkoxycarbonyl, also z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- (C₁-C₆-Alkoxy)carbonyl : (C₁-C₄-Alkoxy)carbonyl, wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dirnethylpropoxycarbonyl, 1,2 -Dimethylpropoxycarbonyl, 1-Methylpentoxycarbonyl, 2-Methylpentoxycarbonyl, 3-Methylpentoxycarbonyl. 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino) carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₆-Alkylamino)carbonyl: (C₁-C₄-Alkylamino) carbonyl, wie vorstehend genannt, sowie z.B. Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, l-Ethyl-l-methylpropylaminocarbonyl oder 1-Ethyl-2-methylpropylaminocarbonyl;
- Di-(C₁-C₄-alkyl)-aminocarbonyl: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N- (1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl N-Ethyl-N-(1,1-dimethylethyl) -aminocarbonyl, N-(1-Methylethyl)-N-propylaminocarbonyl, N-Butyl-N-propylaminocarbonyl, N-(1-Methylpropyl)-N-propylaminocarbonyl, N- (2-Methylpropyl) -N-propylaminocarbonyl, N-(1, 1-Dimethylethyl) -N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N- (3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-M-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1,1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexylaminocarbonyl;
- Di-(C₁-C₆-alkyl)-aminothiocarbonyl: z.B. N.N-Dimethylaminothiocarbonyl, N,N-Diethylaminothiocarbonyl, N,N-Di-(1-methylethyl)aminothiocarbonyl, N,N-Dipropylaminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dirnethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N- (1,1-Dimethylethyl )-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N- (2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1, 2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl) -aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-rnethylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl, N-Pentyl-N-hexylaminothiocarbonyl oder N,N-Dihexylaminothiocarbonyl;
- C₁-C₄-Alkoxy-C₁-C₄-alkyl: durch C₁-C₄-Alkoxy, wie vorstehend genannt, substituiertes C₁-C₄-Alkyl. also z.B. für Methoxymethyl, Ethoxymethyl, Propoxymethyl, (1-Methylethoxy)methyl, Butoxymethyl, (1-Methylpropoxy)methyl, (2-Methylpropoxy)-methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy) propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₄-Alkoxy-C₁-C₄-alkoxy, sowie die Alkoxyalkoxyteile von C₁-C₄-Alkoxy-C₁-C₄-alkoxycarbonyl: durch C₁-C₄-Alkoxy, wie vorstehend genannt, substituiertes C₁-C₄-Alkoxy, also z.B. für Methoxymethoxy, Ethoxymethoxy, Propoxymethoxy, (1-Methylethoxy)methoxy, Butoxymethoxy, (1-Methylpropoxy)methoxy, (2-Methylpropoxy)methoxy, (1,1-Dimethylethoxy)methoxy, 2-(Methoxy) ethoxy, 2-(Ethoxy)ethoxy, 2-(Propoxy)ethoxy, 2-(1-Methylethoxy)ethoxy, 2-(Butoxy)ethoxy, 2- (1-Methylpropoxy)ethoxy, 2-(2-Methylpropoxy)ethoxy, 2-(1,2-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)-propoxy, 3-(2-Methylpropoxy) propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₃-C₆-Alkenyl, sowie die Alkenylteile von C₃-C₆-Alkenylcarbonyl, C₃-C₆-Alkenyloxy, C₃-C₆-Alkenyloxycarbonyl, C₃-C₆-Alkenylaminocarbonyl, N-(C₃-C₆-Alkenyl)-N-(C₁-C₆)alkylaminocarbonyl, N-(C₃-C₆-Alkenyl)-N-(C₁-C₆-alkoxy)aminocarbonyl: z.B. Prop-2-en-1-yl, But-1-en-4-yl, 1-Methylprop-2-en-1-yl, 2-Methyl-prop-2-en-1-yl, 2-Buten-1-yl, 1-Penten-3-yl, 1-Penten-4-yl, 2-Penten-4-yl, 1-Methylbut-2-en-1-yl, 2-Methyl-but-2-en-1-yl, 3-Methylbut-2-en-1-yl, 1-Methyl-but-3-en-1-yl, 2-Methylbut-3-en-1-yl, 3-Methyl-but-3-en-1-yl, 1,1-Dimethylprop-2-en-1-yl, 1,2-Dimethyl-prop-2-en-1-yl, 1-Ethylprop-2-en-1-yl, Hex-3-en-1-yl, Hex-4-en-1-yl, Hex-5-en-1-yl 1-Methyl-pent-3-en-1-yl, 2-Methyl-pent-3-en-1-yl, 3-Methylpent-3-en-1-yl, 4-Methyl-pent-3-en-1-yl, 1-Methylpent-4-en-1-yl, 2-Methyl-pent-4-en-1-yl, 3-Methylpent-4-en-1-yl, 4-Methyl-pent-4-en-1-yl, 1,1-Dimethylbut-2-en-1-yl, 1,1-Dimethyl-but-3-en-1-yl, 1,2-Dimethylbut-2-en-1-yl, 1,2-Dimethyl-but-3-en-1-yl, 1,3-Dimethylbut-2-en-1-yl, 1,3-Dimethyl-but-3-en-1-yl, 2,2-Dimethylbut-3-en-1-yl, 2,3-Dimethyl-but-2-en-1-yl, 2,3-Dimethylbut-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methylprop-2-en-1-yl;
- C₂-C₆-Alkenyl, sowie die Alkenylteile von C₂-C₆-Alkenylcarbonyl, Phenyl-C₂-C₆-alkenylcarbonyl und Heterocyclyl-C₂-C₆-alkenylcarbonyl: C₃-C₆-Alkenyl, wie voranstehend genannt, sowie Ethenyl;
- C₂-C₂ₒ-Alkenyl als Alkenylrest von C₂-C₂₀-Alkenylcarbonyl: C₂-C₆-Alkenyl, wie voranstehend genannt, sowie Hept-6-en-1-yl, Oct-7-en-1-yl, Non-8-en-1-yl, Dec-9-en-1-yl, Dodec-11-en-1-yl, Hexade-15-en-1-yl oder Octadec-17-en-1-yl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinyloxy, C₃-C₆-Alkinyloxycarbony, C₃-C₆-Alkinylaminocarbonyl, N-(C₃-C₆-Alkinyl)-N-(C₁-C₆alkyl)-aminocarbonyl, N-(C₃-C₆-Alkinyl)-N-(C₁-C₆-alkoxyaminocarbonyl: z.B. Propargyl, But-1-in-3-yl, But-1-in-4-yl, But-2-in-1-yl Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-Pent-1-in-4-yl, 3-Methyl-pent-1-in-5-yl, 4-Methyl-pent-2-in-4-yl oder 4-Methyl-pent-2-in-5-yl;
- C₂-C₆-Alkinyl, sowie die Alkinylteile von C₂-C₆-Alkinylcarbonyl: C₃-C₆-Alkinyl, wie voranstehend genannt, sowie Ethinyl;
- C₃-C₆-Halogenalkinyl: einen C₃-C₆-Alkinylrest, wie vorstehend genannt, der partiell oder vollständig durch Fluor, Chlor, Brom und/oder Iod substituiert ist, also z.B. 1,1-Difluorprop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iodbut-3-in-1-yl, 5-Fluorpent-3-in-1-yl, 5-Iod-pent-4-in-1-yl, 6-Fluor-hex-4-in-1-yl oder 6-Iod-hex-5-in-1-yl;
- C₃-C₆-Cycloalkyl, sowie die Cycloalkylteile von C₃-C₆-Cycloalkylcarbonyl: z.B. Cyclopropyl, Cyclobutyl, Cyclopentyl oder Cyclohexyl;
- Heterocyclyl, sowie Heterocyclylteile von Heterocyclyloxy, Heterocyclylcarbonyl, Heterocyclyl-C₁-C₆-alkyl, Heterocyclyloxycarbonyl, Heterocyclyloxythiocarbonyl, Heterocyclyl-C₂-C₆alkenylcarbonyl, Heterocyclylcarbonyl-C₁-C₆-alkyl, N-(C₁-C₆-Alkyl)-N-(heterocyclyl)-aminocarbonyl, Heterocyclylaminocarbonyl: ein gesättigter, partiell gesättigter oder ungesättigter 5- oder 6-gliedriger, C-gebundener, heterocyclischer Ring, der ein bis vier gleiche oder verschiedene Heteroatome, ausgewählt aus folgender Gruppe: Sauerstoff, Schwefel oder Stickstoff, enthält, also z.B. 5-gliedrige Ringe mit einem Heteroatom wie: Tetrahydrofuran-2-yl, Tetrahydrofuran-3-yl, Tetrahydrothien-2-yl, Tetrahydrothien-3-yl,Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 2-Furyl, 3-Furyl, 2-Thienyl, 3-Thienyl, Pyrrol-2-yl oder Pyrrol-3-yl;
   5-gliedrige Ringe mit zwei Heteroatomen wie: Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl oder Thiazol-5-yl;
   5-gliedrige Ringe mit drei Heteroatomen wie: 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadiazolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,3,2-Dioxathiolan-4-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-y1, 1,2,4-Triazolin-3-yl, 3H-1,2,4-Dithiazol-5-yl, 2H-1,3,4-Dithiazol-5-yl, 2H-1,3,4-Oxathiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl oder 1,2,4-Triazol-3-yl;
   5-gliedrige Ringe mit vier Heteroatomen wie: Tetrazol-5-yl,
   6-gliedrige Ringe mit einem Heteroatom wie: Tetrahydropyran-2-yl. Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl. 1,2,3,4-Tetrahydropyridin-4-yl, 1,2,3,4-Tetrahydropyridin-3-yl, 1,2,3,4-Tetrahydropyridin-2-yl, 2H-5, 6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, Pyridin-2-yl, Pyridin-3-yl oder Pyridin-4-yl;
   6-gliedriger Ring mit zwei Heteroatomen wie 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl. 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1, 4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl, Pyridazin-3-yl, Pyridazin-4-yl, Pyrimidin-2-yl, Pyrimidin-4-yl, Pyrimidin-5-yl oder Pyrazin-2-yl;
   6-gliedriger Ring mit drei Heteroatomen wie: 1,3,5-Triazin-2-yl, 1,2,4-Triazin-3-yl, 1,2,4-Triazin-5-yl oder 1,2,4-Triazin-6-yl;
   6-gliedriger Ring mit vier Heteroatomen wie: 1,2,4,5-Tetrazin-3-yl;
wobei ggf. der Schwefel der genannten Heterocyclen zu S=O oder S(=O)₂ oxidiert sein kann
und wobei mit einem ankondensierten Phenylring oder mit einem C₃-C₆-Carboxyclus oder mit einem weiteren 5- bis 6-gliedrigen Heterocyclus ein bicyclisches Ringsystem ausgebildet werden kann.

N-gebundenes Heterocyclyl, sowie die N-gebundenen Heterocyclylteile von O-(N-gebundenes Heterocyclyl): ein gesättigter, partiell gesättigter oder ungesättigter 5- oder 6-gliedriger N-gebundener heterocyclischer Ring, der mindestens einen Stickstoff und gegebenenfalls ein bis drei gleiche oder verschiedene Heteroatome, ausgewählt aus folgender Gruppe: Sauerstoff, Schwefel oder Stickstoff enthält, also z.B.

N-gebundene 5-gliedrige Ringe wie:
Tetrahydropyrrol-1-yl, 2,3-Dihydro-1H-pyrrol-1-yl, 2,5-Dihydro-1H-pyrrol-1-yl, Pyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, Pyrazol-1-yl, Imidazol-1-yl. 1,2,4-Δ⁴-Oxaiazolin-2-yl, 1,2,4-Δ²-Oxadiazolin-4-yl, 1,2,4-Δ³-Oxadiazolin-2-yl, 1,3,4-Δ²-Oxadiazolin-4-yl, 1,2,4-Δ⁵-Thiadiazolin-2-yl, 1,2,4-Δ³-Thiadiazolin-2-yl, 1,2,4-Δ²-Thiadiazolin-4-yl, 1,3,4-Δ²-Thiadiazolin-4-yl, 1,2,3-Δ²-Triazolin-1-yl, 1,2,4-Δ²-Triazolin-1-yl, 1,2,4-Δ²-Triazolin-4-yl, 1,2,4-Δ³-Triazolin-1-yl, 1,2,4-Δ¹-Triazolin-4-yl, 1,2,3-triazol-1-yl, 1,2,4-Triazol-1-yl, tetrazol-1-yl;
sowie N-gebundene 6-gliedrige Ringe wie:
Piperidin-1-yl, 1,2,3,4-Tetrahydropyridin-1-yl, 1,2,5,6-Tetrahydropyridin-1-yl, 1,4-Dihydropyridin-1-yl, 1,2-Dihydropyridin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazinoxazin-2-yl, 2H-3,4-Dihydro-1,2-thiazin-2-yl, 2,3,4,5-Tetrahydropyridazin-2-yl, 1,2,5,6-Tetrahydropyridazin-l-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl;
sowie N-gebundene cyclische Imide wie:
Phthalsäureimid, Tetrahydrophthalsäureimid, Succinimid, Maleinimid, Glutarimid, 5-Oxo-triazolin-1-yl, 5-Oxo-1,3,4-oxadiazolin-4-yl oder 2,4-Dioxo-(1H,3H)-pyrimidin-3-yl;
wobei mit einem ankondensierten Phenylring oder mit einem C₃-C₆-Carbocyclus oder einem weiteren 5- bis 6-gliedrigen Heterocyclus ein bicyclisches Ringsystem ausgebildet werden kann.

Alle Phenylringe bzw. Heterocyclylreste sowie alle Phenylkomponenten in Phenoxy, Phenyl-C₁-C₆-alkyl, Phenylcarbonyl-C₁-C₆-alkyl, Phenylcarbonyl, Phenylalkenylcarbonyl, Phenoxycarbonyl, Phenyloxythiocarbonyl, Phenylaminocarbonyl und N-(C₁-C₆-Alkyl)-N-phenylaminocarbonyl bzw. Heterocyclylkomponenten in Heterocyclyloxy, Heterocyclyl-C₁-C₆-alkyl, Heterocyclylcarbonyl-C₁-C₆-alkyl, Heterocyclylcarbonyl, Heterocyclyloxythiocarbonyl, Heterocyclylalkenylcarbonyl, Heterocyclyloxycarbonyl, Heterocyclylaminocarbonyl und N(C₁-C₆-Alkyl)-N-heterocyclylaminocarbonyl sind, soweit nicht anders angegeben, vorzugsweise unsubstituiert oder tragen ein bis drei Halogenatome und/oder eine Nitrogruppe, einen Cyanorest und/ oder einen oder zwei Methyl-, Trifluormethyl-, Methoxy- oder Trifluormethoxysubstituenten.

Die erfindungsgemäßen Verbindungen der Formel I mit R⁷ = IIa werden als Verbindungen der Formel Ia bezeichnet.

Von besonderer Bedeutung sind die erfindungsgemäßen Verbindungen der Formel I, wobei
- R¹¹: C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₂₀-Alkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Phenyloxythiocarbonyl, Heterocyclyloxycarbonyl, Heterocyclyloxythiocarbonyl, Phenylaminocarbonyl, N-(C₁-C₆-Alkyl)-N-(phenyl)-aminocarbonyl, Heterocyclylaminocarbonyl, N-(C₁-C₆-Alkyl)-N-(heterocyclyl)-aminocarbonyl, Phenyl-C₂-C₆-alkenylcarbonyl oder Heterocyclyl-C₂-C₆-alkenylcarbonyl, wobei der Phenyl- und der Heterocyclyl-Rest der 18 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Heterocyclyl oder N-gebundenes Heterocyclyl, wobei die beiden letztgenannten Substituenten ihrerseits partiell oder vollständig halogeniert sein können und/oder einen bis drei der folgenden Reste tragen können: Nitro, Cyano, C₁-C₄-Alkyl, C₁-C₄-Halogenalkyl, C₁-C₄-Alkoxy oder C₁-C₄-Halogenalkoxy;
bedeutet.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl oder C₁-C₆-Halogenalkylsulfonyl;
- R³: Wasserstoff;
- R⁴, R⁵: Wasserstoff, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl oder C₁-C₆-Halogenalkylsulfonyl;
oder
- R⁴ und R⁵: bilden gemeinsam eine -O-(CH₂)ₘ-O-, -O-(CH₂)ₘ-S-, - S-(CH₂)ₘ-S- oder -O-(CH₂)ₙ-Kette, die durch einen bis drei Reste aus folgender Gruppe substituiert sein kann: Halogen, Cyano, C₁-C₄-Alkyl, C₁-C₄-Halogenalkyl oder C₁-C₄-Alkoxycarbonyl;
oder
- R⁴ und R⁵: bilden gemeinsam eine -(CH₂)ₚ-Kette, die durch Sauerstoff oder Schwefel unterbrochen sein kann und/oder durch einen bis vier Reste aus folgender Gruppe substituiert sein kann:
Halogen, Cyano, C₁-C₄-Alkyl, C₁-C₄-Halogenalkyl oder C₁-C4-Alkoxycarbonyl;
oder
- R⁴ und R⁵: bilden gemeinsam eine Methylidengruppe, die durch einen bis zwei Reste aus folgender Gruppe substituiert sein kann:
Halogen, Cyano, C₁-C₆-Alkyl, C₁-C₆-Halogenalkyl, C₁-C₆-Alkoxy oder C₁-C₆-Halogenalkoxy;
- l: 0;
- m: 2 bis 4;
- n: 1 bis 5;
- p: 2 bis 5;
- R⁷: eine Verbindung IIa 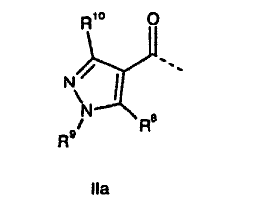
wobei
- R⁸: Halogen, OR¹¹, SR¹¹, SO₂R¹², POR¹²R¹³, OPOR¹²R¹³, OPSR¹²R¹³, NR¹⁴R¹⁵, ONR¹⁵R¹⁵, N-gebundenes Heterocyclyl oder O-(N-gebundenes Heterocyclyl), wobei der Heterocyclyl-Rest der beiden letztgenannten Substituenten partiell oder vollständig halogeniert sein kann und/ oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R⁹: Wasserstoff, C₁-C₆-Alkyl oder C₁-C₆-Halogenalkyl;
- R¹⁰: Wasserstoff, C₁-C₆-Alkyl oder C₁-C₆-Halogenalkyl;
- R¹¹: C₁-C₆-Alkyl, C₃-C₆-Alkenyl, C₃-C₆-Halogenalkenyl, C₃-C₆-Alkinyl, C₁-C₂₀-Alkylcarbonyl, C₂-C₂₀-Alkenylcarbonyl, C₃-C₆-Cycloalkylcarbonyl, (2-Norbornyl)-methylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl) N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkylund Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Phenyloxythiocarbonyl, Heterocyclyloxycarbonyl, Heterocyclyloxythiocarbonyl, Phenyl-C₂-C₆-alkenylcarbonyl oder Heterocyclyl-C₂-C₆-alkenylcarbonyl, wobei der Phenyl- und der HeterocyclylRest der 14 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Phenoxy, Heterocyclyl oder N-gebundenes Heterocyclyl, wobei die drei letztgenannten Substituenten ihrerseits partiell oder vollständig halogeniert sein können und/oder einen bis drei der folgenden Reste tragen können: Nitro, Cyano, C₁-C₄-Alkyl, C₁-C₄-Halogenalkyl, C₁-C₄-Alkoxy oder C₁-C₄-Halogenalkoxy;
- R¹², R¹³: C₁-C₆-Alkyl, C₃-C₆-Alkenyl, C₃-C₆-Halogenalkenyl, C₃-C₆-Cycloalkyl, Hydroxy, C₁-C₆-Alkoxy oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl) -aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁴: C₁-C₆-Alkyl, C₃-C₆-Alkenyl, C₃-C₆-Halogenalkenyl, C₃-C₆-Cycloalkyl, C₁-C₆-Alkoxy, C₃-C₆-Alkenyloxy oder Di-(C₁-C₆-Alkyl)-amino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁵: C₁-C₆-Alkyl oder C₃-C₆-Alkenyl;

Von besonderer Bedeutung sind hierbei die erfindungsgemäßen Verbindungen der Formel I, wobei
- R¹¹: C₁-C₆-Alkyl, C₃-C₆-Alkenyl, C₃-C₆-Halogenalkenyl, C₃-C₆-Alkinyl, C₁-C₂₀-Alkylcarbonyl, C₂-C₆-Alkenylcarbonyl, C₃-C₆-Cycloalkylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N- (C₁-C₆-Alkylamino) -imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Phenyloxythiocarbonyl, Heterocyclyloxycarbonyl, Heterocyclyloxythiocarbonyl, Phenyl-C₂-C₆-alkenylcarbonyl oder Heterocyclyl-C₂-C₆-alkenylcarbonyl, wobei der Phenyl- und der HeterocyclylRest der 14 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Phenoxy, Heterocyclyl oder N-gebundenes Heterocyclyl, wobei die drei letztgenannten Substituenten ihrerseits partiell oder vollständig halogeniert sein können und/oder einen.bis drei der folgenden Reste tragen können: Nitro, Cyano, C₁-C₄-Alkyl, C₁-C₄-Halogenalkyl, C₁-C₄-Alkoxy oder C₁-C₄-Halogenalkoxy;

Besonders bevorzugt sind Verbindungen der Formel I, wobei die Variablen folgende Bedeutungen haben, und zwar für sich allein oder in Kombination:
- R¹: Halogen, C₁-C₆-Alkyl, C₁-C₆-Halogenalkyl, C₁-C₆-Alkoxy, C₁-C₆-Alkylthio oder C₁-C₆-Alkylsulfonyl;
insbesondere Halogen wie Chlor oder Brom, C₁-C₆-Alkyl wie Methyl oder Ethyl oder C₁-C₆-Alkoxy wie Methoxy oder Ethoxy;
besonders bevorzugt Chlor, Methyl oder Methoxy;
- R³: Wasserstoff;
- R⁴, R⁵: Wasserstoff, C₁-C₆-Alkyl, C₁-C₆-Halogenalkyl, C₁-C₆-Alkoxy oder C₁-C₆-Halogenalkoxy; insbesondere Wasserstoff, C₁-C₆-Alkyl oder C₁-C₆-Alkoxy;
besonders bevorzugt Wasserstoff oder C₁-C₆-Alkyl wie Methyl oder Ethyl;
oder
- R⁴ und R⁵: bilden gemeinsam eine -O-(CH₂)ₘ-O-, -O-(CH₂)ₘ-S- oder -S-(CH₂)ₘ-S-Kette, die durch einen bis drei Reste aus folgender Gruppe substituiert sein kann:
C₁-C₄-Alkyl oder C₁-C₄-Halogenalkyl;
oder
- R⁴ und R⁵: bilden gemeinsam eine-(CH₂)ₚ-Kette, die durch einen bis vier Reste aus folgender Gruppe substituiert sein kann:
Halogen, C₁-C₄-Alkyl oder C₁-C₄-Halogenalkyl;
oder
- R⁴ und R⁵: bilden gemeinsam eine Methylidengruppe, die durch einen bis zwei Reste aus folgender Gruppe substituiert sein kann:
Halogen, Cyano, C₁-C₆-Alkyl, C₁-C₆-Halogenalkyl, C₁-C₆-Alkoxy oder C₁-C₆-Halogenalkoxy;
- l: 0;
- m: 2 bis 4;
insbesondere 2 oder 3
- p: 2 bis 5;
- R⁷: eine Verbindung IIa 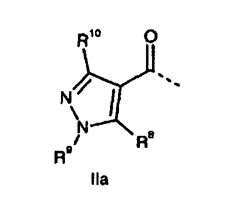
wobei
- R⁸: Halogen, OR¹¹, SR¹¹, SO₂R^{12,} NR¹⁴R¹⁵, ONR¹⁵R¹⁵, N-gebundenes Heterocyclyl oder O-(N-gebundenes Heterocyclyl), wobei der Heterocyclyl-Rest der beiden letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R⁹: C₁-C₆-Alkyl;
- R¹⁰: Wasserstoff oder C₁-C₆-Alkyl;
- R¹¹: C₁-C₆-Alkyl, C₃-C₆-Alkenyl, C₃-C₆-Halogenalkenyl, C₃-C₆-Alkinyl, C₁-C₂₀-Alkylcarbonyl, C₃-C₆-Cycloalkylcarbonyl, C₁-C₆-Alkoxycarbonyl, C₃-C₆-Alkenyloxycarbonyl, C₁-C₆-Alkylaminocarbonyl, C₃-C₆-Alkenylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, Di-(C₁-C₆-alkyl)-aminothiocarbonyl oder C₁-C₆-Alkylcarbonyl-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyl, Heterocyclylcarbonyl-C₁-C₆-alkyl, Phenylcarbonyl, Heterocyclylcarbonyl, Phenoxycarbonyl, Phenyloxythiocarbonyl, Heterocyclyloxycarbonyl oder Heterocyclyloxythiocarbonyl, wobei der Phenyl- und der Heterocyclyl-Rest der 12 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy Heterocyclyl oder N-gebundenes Heterocyclyl, wobei die beiden letztgenannten Substituenten ihrersetis partiell oder vollständig halogeniert sein können und/oder einen bis drei der folgenden Reste tragen können: Nitro, Cyano, C₁-C₄-Alkyl, C₁-C₄-Halogenalkyl, C₁-C₄-Alkoxy oder C₁-C₄-Halogenalkoxy;
- R¹², R¹³: C₁-C₆-Alkyl, C₃-C₆-Alkenyl, C₃-C₆-Halogenalkenyl, C₃-C₆-Cycloalkyl, Hydroxy, C₁-C₆-Alkoxy oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl)-aminocarbonyl, C₁-C₄-Alkylcarbonyloxy oder C₃-C₆-Cycloalkyl;
Phenyl, Heterocyclyl, phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁴: C₁-C₆-Alkyl, C₃-C₆-Alkenyl, C₃-C₆-Halogenalkenyl, C₃-C₆-Cycloalkyl, C₁-C₆-Alkoxy, C₃-C₆-Alkenyloxy oder Di-(C₁-C₆-Alkyl)-amino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio, C₁-C₄-Alkylcarbonyl, C₁-C₄-Alkoxycarbonyl, Hydroxycarbonyl, Di-(C₁-C₄-alkyl) -aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
- R¹⁵: C₁-C₆-Alkyl oder C₃-C₆-Alkenyl;

Insbesondere bevorzugt sind Verbindungen der Formel I, wobei
- R⁴: Wasserstoff, C₁-C₆-Alkyl, C₁-C₆-Halogenalkyl, C₁-C₆-Alkoxy oder C₁-C₆-Halogenalkoxy;
besonders bevorzugt Wasserstoff, C₁-C₆-Alkyl wie Methyl oder Ethyl oder C₁-C₆-Alkoxy wie Methoxy oder Ethoxy;
- R⁵: Wasserstoff oder C₁-C₆-Alkyl; besonders bevorzugt Wasserstoff oder Methyl;
bedeuten
oder
- R⁴ und R⁵: bilden gemeinsam eine -O-(CH₂)₂-O-, -O-(CH₂)₃-O-, -O-(CH₂)₂-S-, -O-(CH₂)₃-S-, -S-(CH₂)₂-S-, -S-(CH₂)₃-S-, -(CH₂)₂-, -(CH₂)₄ oder (CH₂)₅-Kette, die durch einen bis drei C₁-C₄-Alkyl- oder C₁-C₄-Halogenalkyl-Reste substituiert sein kann;
oder
- R⁴ und R⁵: bilden gemeinsam eine Methylidengruppe, die durch einen Rest der folgenden Gruppe substituiert sein kann: Halogen wie Chlor oder Brom, C₁-C₆-Alkyl wie Methyl oder Ethyl, C₁-C₆-Halogenalkyl wie Chlormethyl, Fluormethyl, Dichlormethyl, Difluormethyl oder Trifluormethyl, C₁-C₆-Alkoxy wie Methoxy oder Ethoxy.

Insbesonderst bevorzugt sind die Verbindungen der Formel I, wobei
- R⁴: Wasserstoff, C₁-C₆-Alkyl, C₁-C₆-Halogenalkyl, C₁-C₆-Alkoxy oder C₁-C₆-Halogenalkoxy;
besonders bevorzugt Wasserstoff, C₁-C₆-Alkyl wie Methyl oder Ethyl oder C₁-C₆-Alkoxy wie Methoxy oder Ethoxy;
- R⁵: Wasserstoff oder C₁-C₆-Alkyl;
besonders bevorzugt Wasserstoff oder Methyl;
bedeuten.

Ebenso insbesondere bevorzugt sind die Verbindungen der Formel I, wobei
- R⁸: NR¹⁴R¹⁵ oder N-gebundenes Heterocyclyl, das partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
bedeutet.

Insbesonderst bevorzugt sind Verbindungen der Formel I, wobei
- R⁸: NR¹⁴R¹⁵ oder Tetrahydropyrrol-1-yl, 2,3-Dihydro-1H-pyrrol-1-y 2,5-Dihydro-1H-pyrrol-1-yl, Pyrrol-1-yl, Tetrahydropyrazol-1-yl, Tetrahydroisoxazol-2-yl, Tetrahydrothiazol-2-yl, Tetrahydroimidazol-1-yl, Tetrahydrooxazol-3-yl, Tetrahydrothiazol-3-yl, Pyrazol-1-yl, Imidazol-1-yl, 1,2,4-Triazol-1-y Tetrazol-1-yl, Piperidin-1-yl, Hexahydropyrimidin-1-yl, Hexahydropyrazin-1-yl, Tetrahydro-1,4-oxazin-4-yl, Tetrahydro-1,2-oxazin-2-yl, Succinimid, Maleinimid oder Glutarimid, wobei die genannten Heterocyclen partiell oder vollständig halogeniert sein können und/oder einen bis drei der folgenden Reste tragen können:
Nitro, Cyano, C₁-C₄-Alkyl, wie Methyl oder Ethyl, C₁-C₄-Halogenalkyl wie Chlormethyl, Difluormethyl oder Trifluormethyl, C₁-C₄-Alkoxy wie Methoxy oder Ethoxy oder C₁-C₄-Halogenalkoxy wie Difluormethoxy oder Trifluormethoxy;

Ebenso insbesondere bevorzugt sind die Verbindungen der Formel I, wobei R⁸ für OR¹¹ steht.

Insbesonders bevorzugt sind die Verbindungen der Formel I, wobei R⁸ für OR¹¹ steht und
- R¹¹: C₁-C₂₀-Alkylcarbonyl, C₂-C₂₀-Alkenylcarbonyl, C₂-C₆-Alkinylcarbonyl, C₃-C₆-Cycloalkylcarbonyl oder (2-Norbornyl)methylcarbonyl, wobei die genannten Alkylund Cycloalkylreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-amino-carbonyl, Aminocarbonyl, C₁-C₄-Alkylcarbonyloxy oder C₃-C₆-Cycloalkyl;
Phenylcarbonyl-C₁-C₆-alkyl, Heterocyclylcarbonyl-C₁-C₆-alkyl, Phenylcarbonyl oder Heterocyclylcarbonyl, wobei der Phenyl- und der Heterocyclyl-Rest der 4 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Phenoxy, Heterocyclyl oder N-gebundenes Heterocyclyl, wobei die drei letztgenannten Substituenten ihrerseits partiell oder vollständig halogeniert sein können und/oder einen bis drei der folgenden Reste tragen können:
Nitro, Cyano, C₁-C₄-Alkyl, C₁-C₄-Halogenalkyl, C₁-C₄-Alkoxy oder C₁-C₄-Halogenalkoxy;
bedeutet.

Besondere Bedeutung haben die Verbindungen der Formel I, wobei R⁸ für OR¹¹ steht und
- R¹¹: C₁-C₂₀-Alkylcarbonyl, C₂-C₆-Alkenylcarbonyl, C₂-C₆-Alkinylcarbonyl oder C₃-C₆-Cycloalkylcarbonyl, wobei die genannten Alkyl- und Cycloalkylreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-amino-carbonyl, Aminocarbonyl, C₁-C₄-Alkylcarbonyloxy oder C₃-C₆-Cycloalkyl;
Phenylcarbonyl-C₁-C₆-alkyl, Heterocyclylcarbonyl-C₁-C₆-alkyl, Phenylcarbonyl oder Heterocyclylcarbonyl, wobei der Phenyl- und der Heterocyclyl-Rest der 4 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Heterocyclyl oder N-gebundenes Heterocyclyl, wobei die beiden letztgenannten Substituenten ihrerseits partiell oder vollständig halogeniert sein können und/oder einen bis drei der folgenden Reste tragen können:
   Nitro, Cyano, C₁-C₄-Alkyl, C₁-C₄-Halogenalkyl, C₁-C₄-Alkoxy oder C₁-C₄-Halogenalkoxy;
bedeutet.

Weiterhin sind die Verbindungen I insbesondere bevorzugt, wobei
- R¹: Halogen oder C₁-C₆-Alkyl;
insbesondere C₁-C₄-Alkyl wie Methyl oder Ethyl;
- R³: Wasserstoff;
- R⁴: Wasserstoff oder C₁-C₆-Alkyl;
insbesondere C₁-C₄-Alkyl wie Methyl oder Ethyl;
- R⁵: Wasserstoff oder C₁-C₆-Alkyl;
insbesondere C₁-C₄-Alkyl wie Methyl oder Ethyl;
- l: 0;
- R⁷: eine Verbindung IIa;
- R⁸: Halogen, wie Chlor oder Brom, oder OR¹¹;
- R⁹: C₁-C₆-Alkyl;
insbesondere C₁-C₄-Alkyl;
- R¹⁰: Wasserstoff oder C₁-C₆-Alkyl;
insbesondere Wasserstoff oder C₁-C₄-Alkyl wie Methyl oder Ethyl;
- R¹¹: C₁-C₂₀-Alkylcarbonyl, C₂-C₂₀-Alkenylcarbonyl oder
(2-Norbornyl)methylcarbonyl, wobei der Alkylrest partiell oder vollständig halogeniert sein kann und/oder eine bis drei der folgenden Gruppen tragen kann:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, Hydroxycarbonyl, C₁-C₄-Alkylaminocarbonyl, Di-(C₁-C₄-alkyl)aminocarbonyl, Aminocarbonyl oder C₃-C₆-Cycloalkyl;
Phenylcarbonyl-C₁-C₆-alkyl, Phenylcarbonyl oder Heterocyclylcarbonyl, wobei der Phenyl- und der Heterocyclylrest der drei letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, Phenoxy, Heterocyclyl oder N-gebundenes Heterocyclyl, wobei die drei letztgenannten Substituenten ihrerseits partiell oder vollständig halogeniert sein können und/oder einen bis drei der folgenden Reste tragen können:
   Nitro, Cyano, C₁-C₄-Alkyl, C₁-C₄-Halogenalkyl, C₁-C₄-Alkoxy oder C₁-C₄-Halogenalkoxy;

Außerordentlich bevorzugt sind die Verbindungen der Formel Ia1 und Ib1(≡ I mit X = C(CH₃)₂ und l = 0), insbesondere die Verbindungen Ia1.1 bis Ia1.522 und die Verbindungen Ib1.1 bis Ib1.52 wobei die Restedefinitionen R¹ bis R¹⁰ und l nicht nur in Kombination miteinander, sondern auch für sich allein betrachtet für die erfindungsgemäßen Verbindungen eine besondere Bedeutung haben.

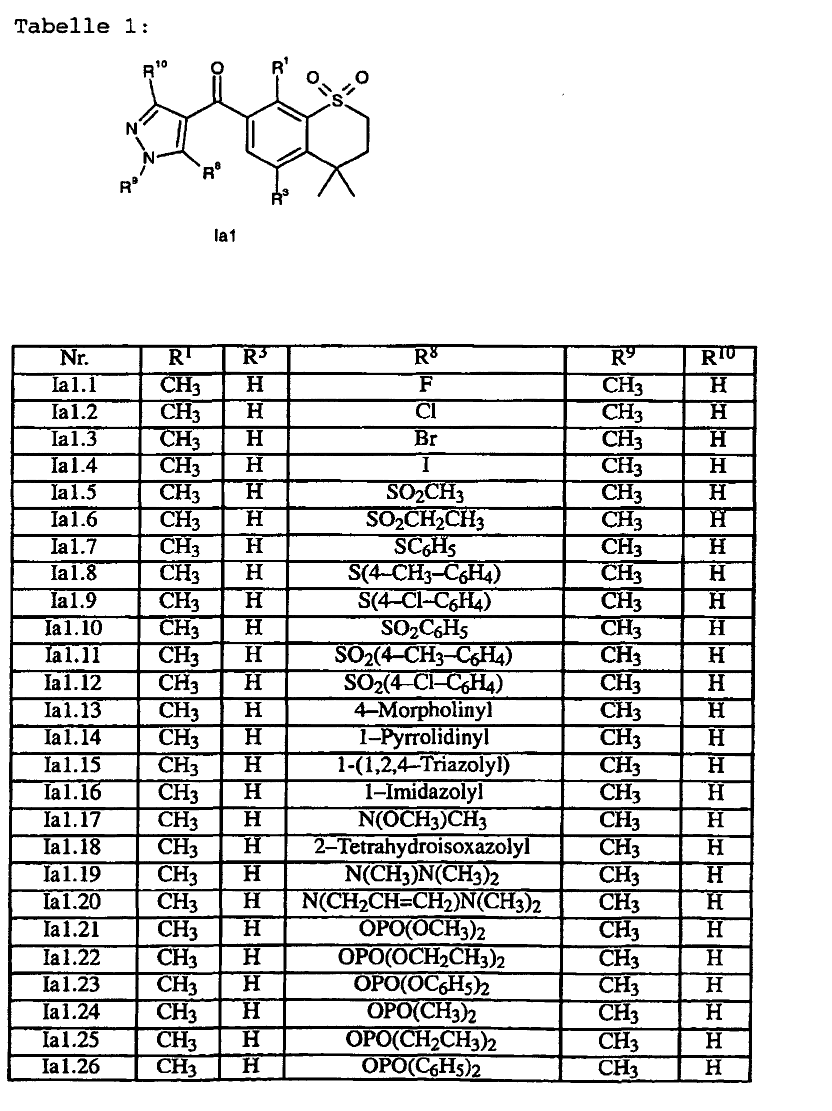

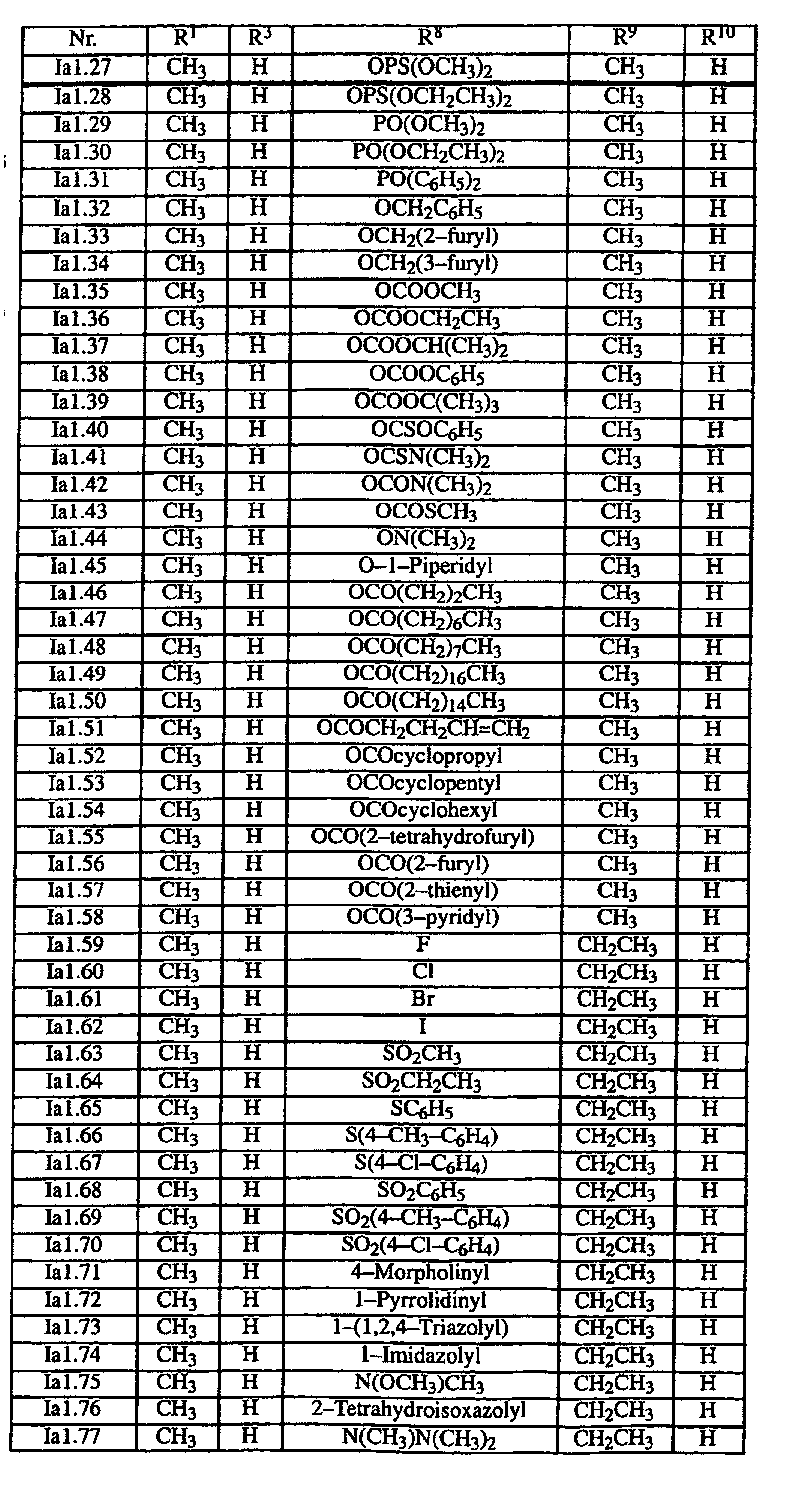

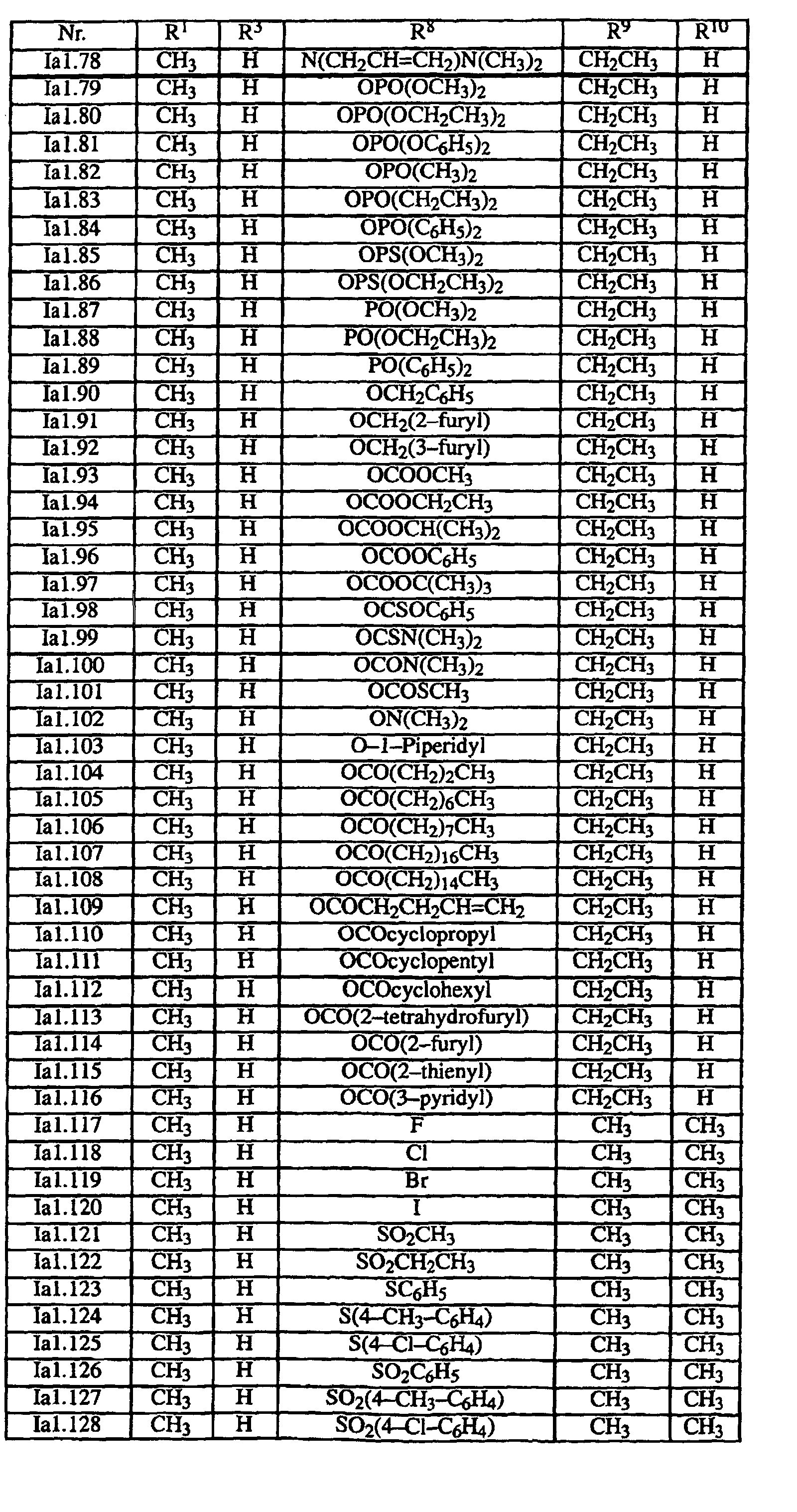

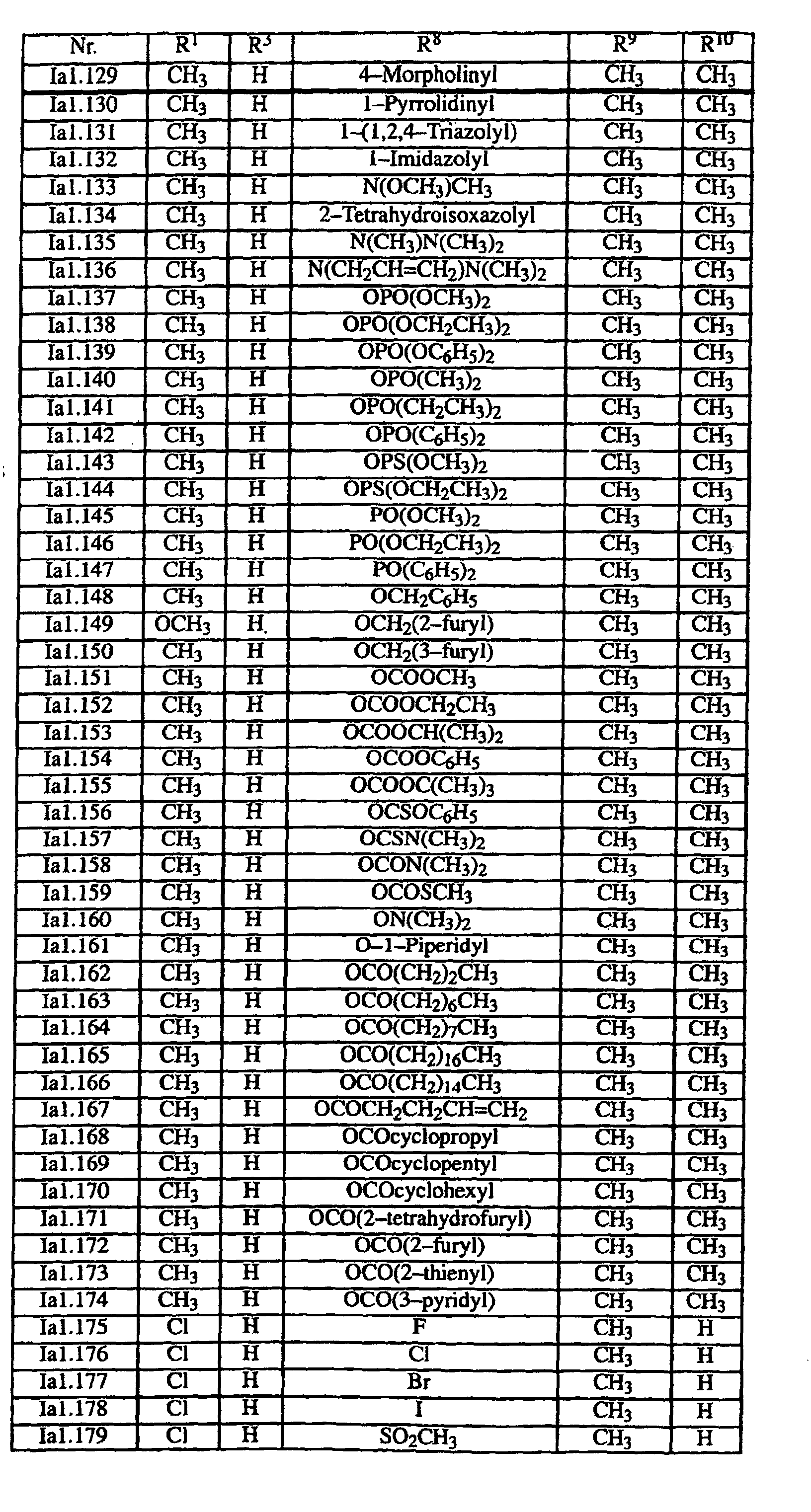

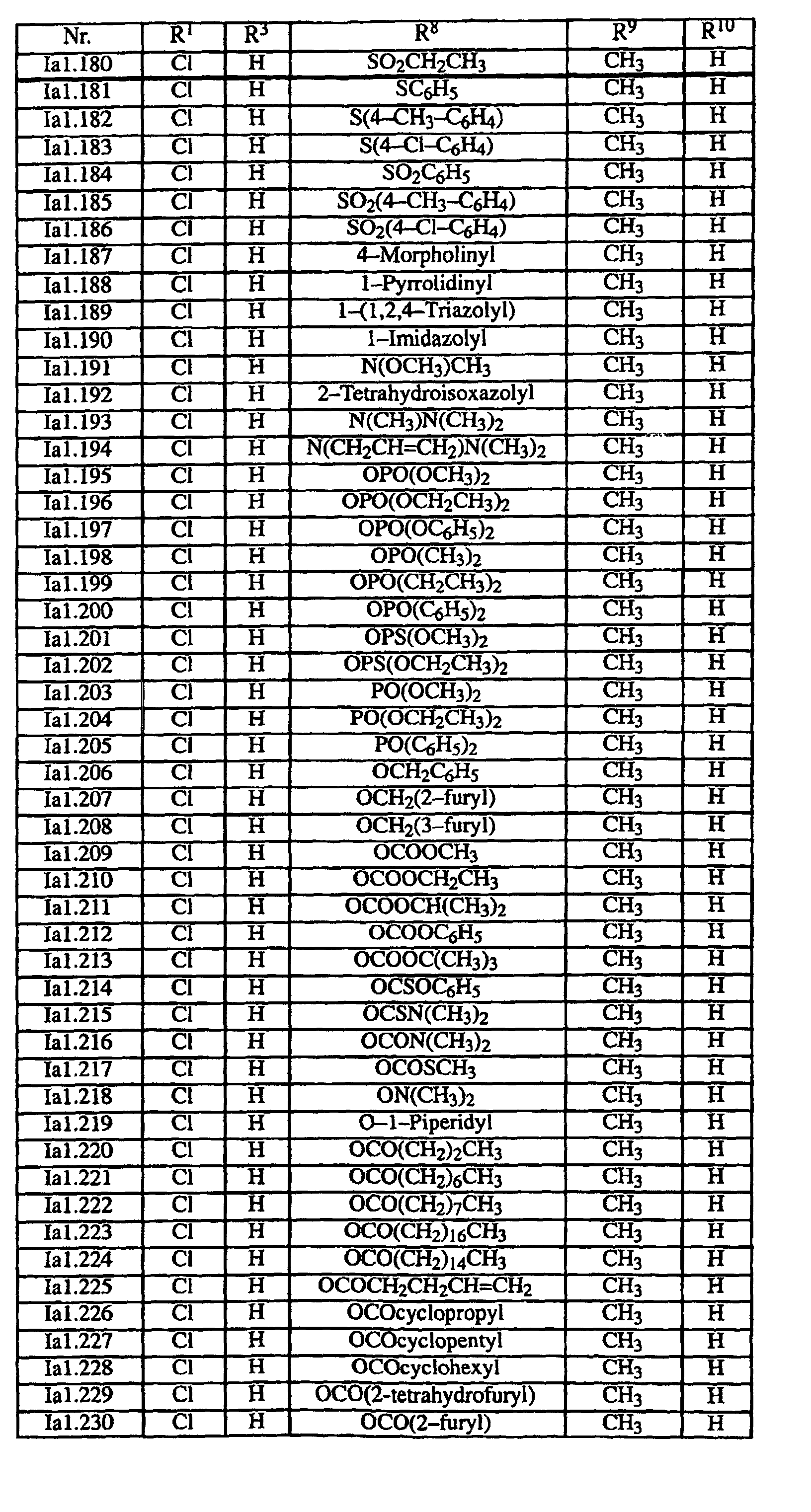

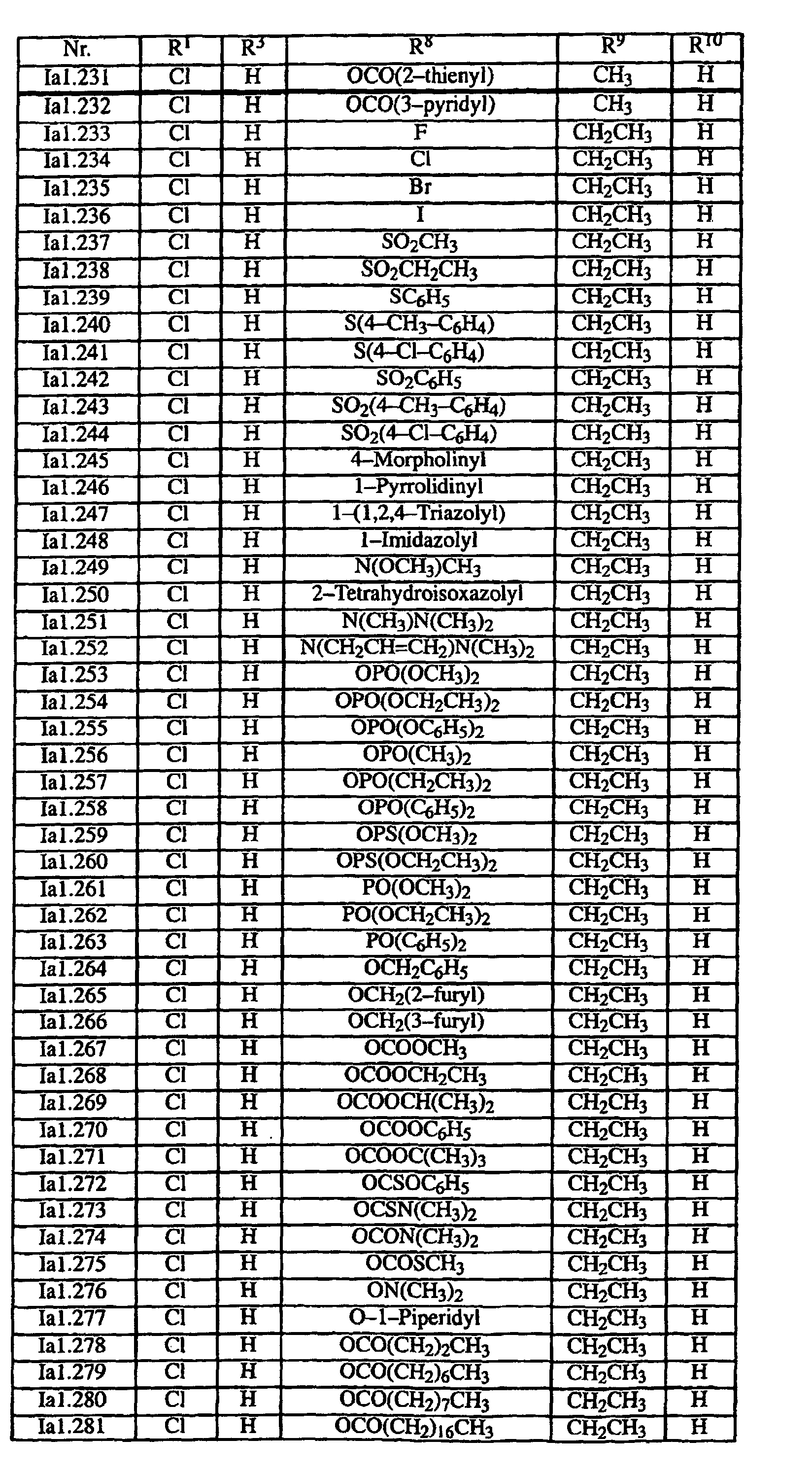

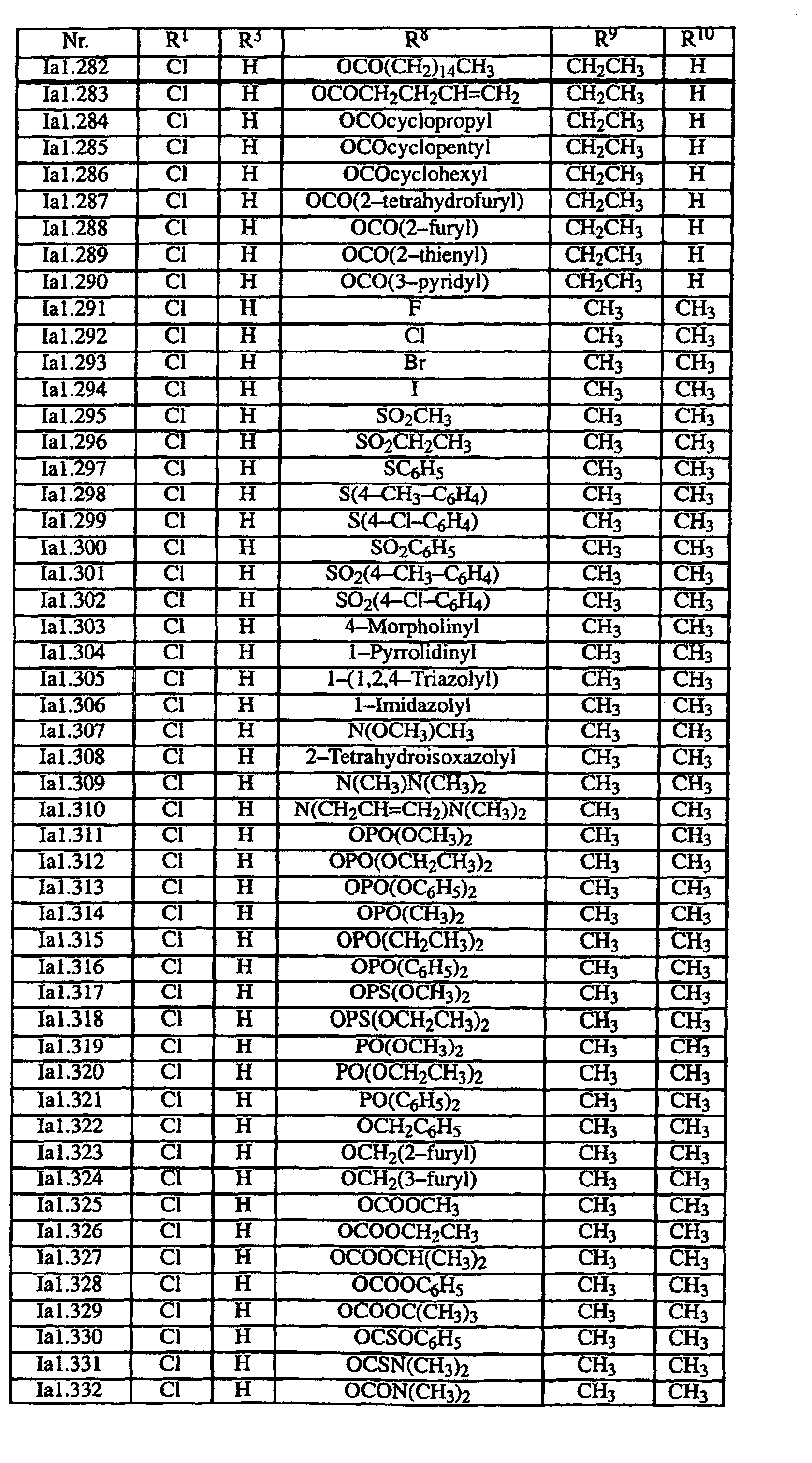

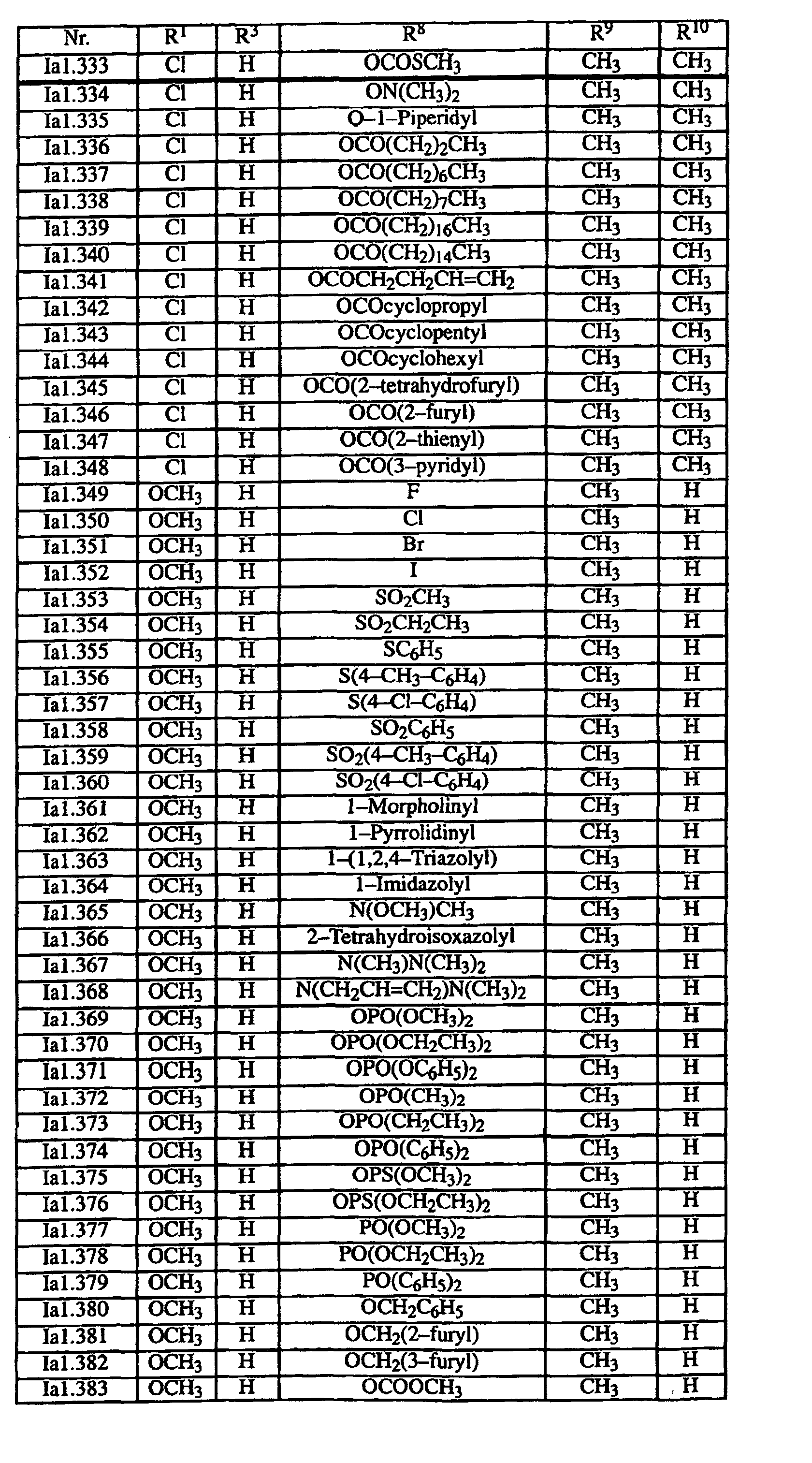

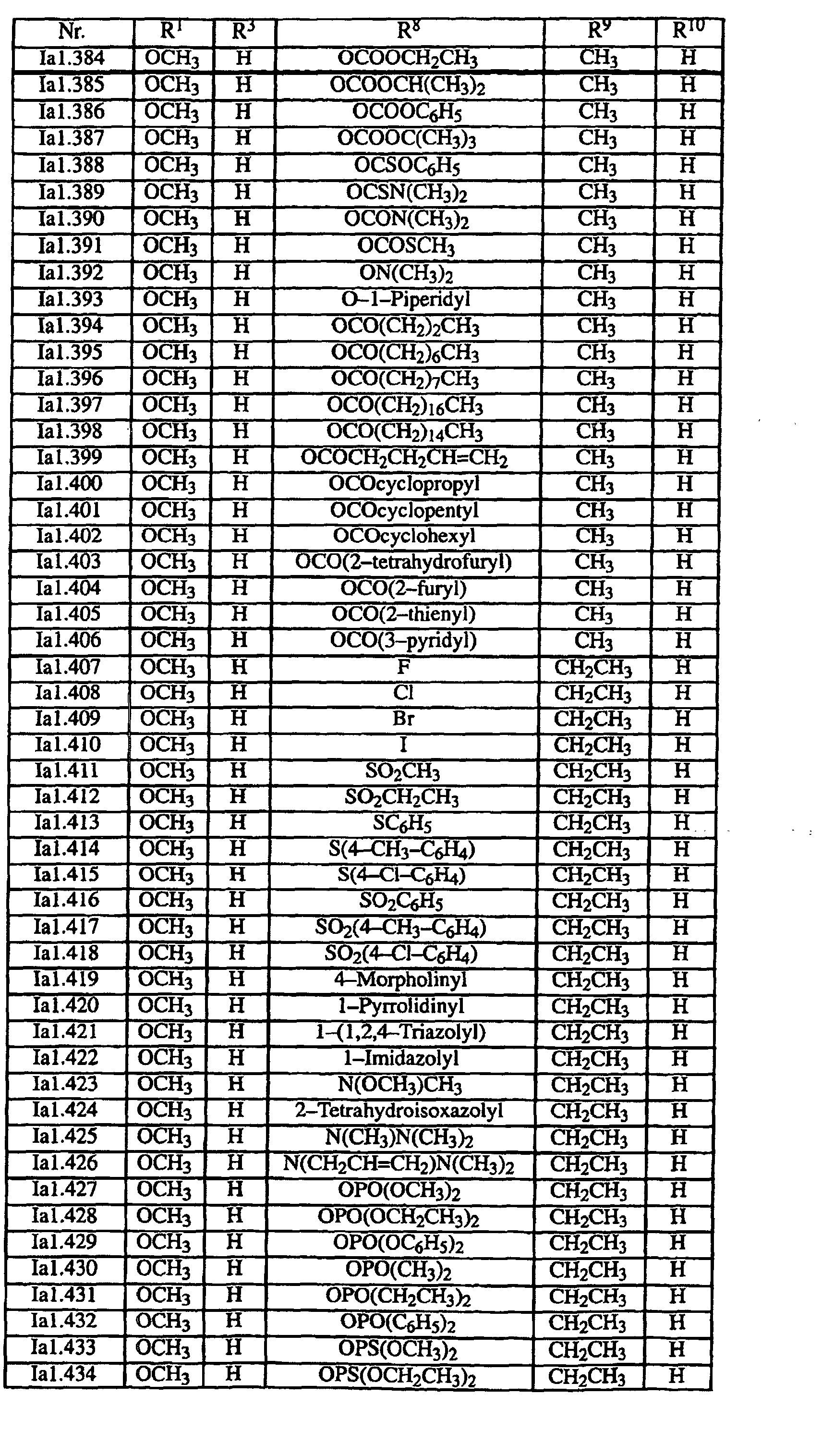

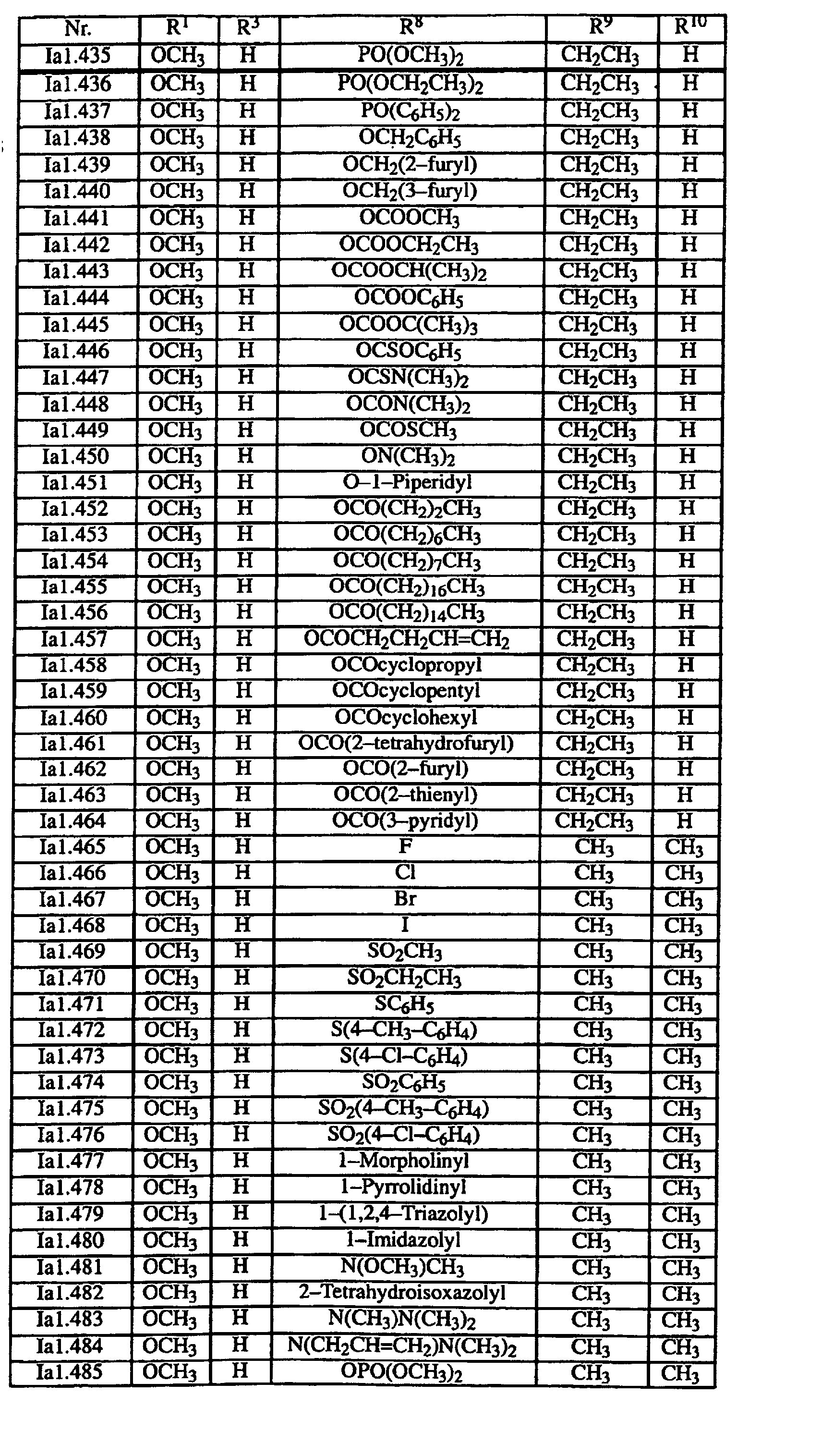

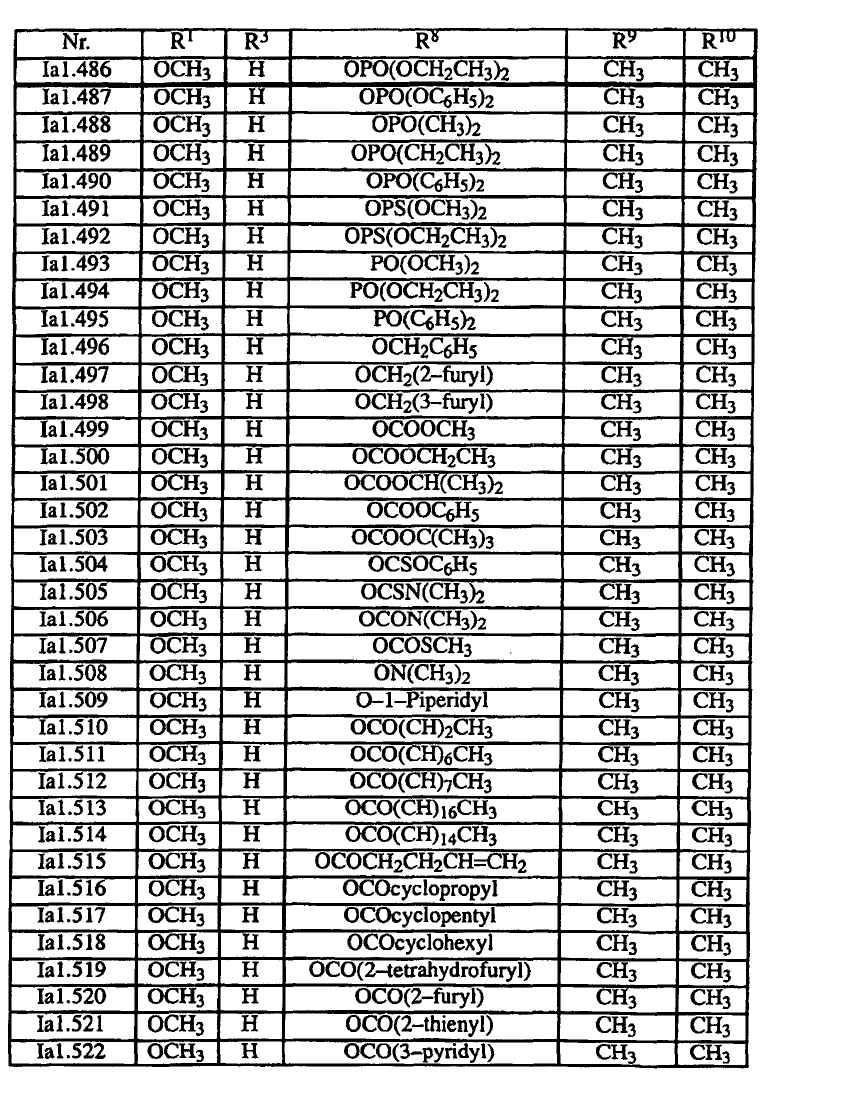

Desweiteren sind folgende Pyrazolyldioxothiochromanoyl-Derivate der Formel I außerordentlich bevorzugt:
- Die Verbindungen der Formel Ia2, insbesondere die Verbindungen Ia2.1 bis Ia2.522, die sich von den Verbindungen Ia1.1 bis Ia1.522 dadurch unterscheiden, daß x CH(CH₃) bedeutet. 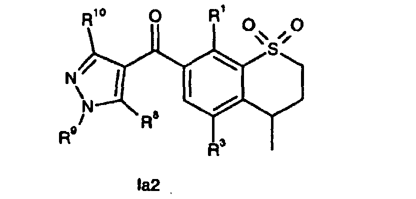
- Die Verbindungen der Formel Ia3, insbesondere die Verbindungen Ia3.1 bis Ia3.522, die sich von den Verbindungen Ia1.1 bis Ia1.522 dadurch unterscheiden, daß X CH(OCH₃) bedeutet. 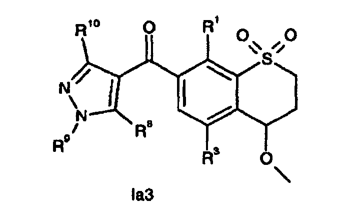
- Die Verbindungen der Formel Ia4, insbesondere die Verbindungen Ia4.1 bis Ia4.522, die sich von den Verbindungen Ia1.1 bis Ia1.522 dadurch unterscheiden, daß X C(CH₃)(OCH₃) bedeutet. 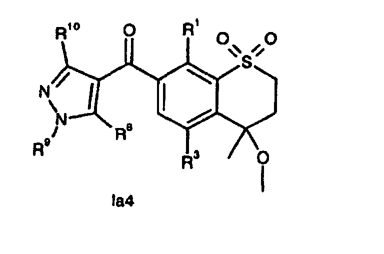
- Die Verbindungen der Formel Ia7, insbesondere die Verbindungen Ia7.1 bis Ia7.522, die sich von den Verbindungen Ia1.1 bis Ia1.522 dadurch unterscheiden, daß X C(OCH₃)₂ bedeutet. 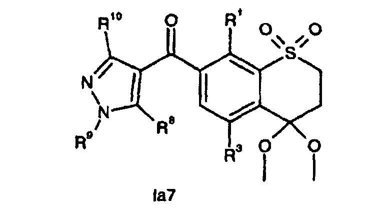
- Die Verbindungen der Formel Ia8, insbesondere die Verbindungen Ia8.1 bis Ia8.522, die sich von den Verbindungen Ia1.1 bis Ia1.522 dadurch unterscheiden, daß X S(=O)₂ bedeutet.

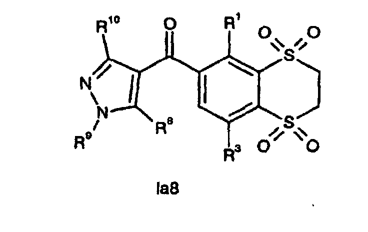

Die Pyrazolyldioxothiochromanoyl-Derivate der Formel I sind auf verschiedene Art und Weise erhältlich, beispielsweise nach folgenden Verfahren:
A. Darstellung von Verbindungen der Formel I mit R⁸ = Halogen durch Umsetzung von Pyrazolon-Derivaten der Formel III mit Halogenierungsmitteln: 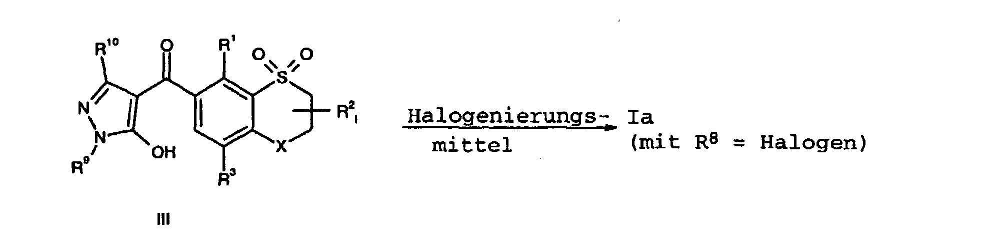
   Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxylylbromid, Phosphoroxybromid etc.
   Die Ausgangsverbindungen werden in der Regel im äquimolaren Verhältnis eingesetzt. Es kann aber auch von Vorteil sein, die eine oder andere Komponente im Überschuß einzusetzen.
   Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, polare aprotische Lösungsmittel, wie Acetonitril, Dimethylformamid oder Dimethylsulfoxid oder Gemische hiervon in Betracht. Die Reaktion kann auch in Substanz durchgeführt werden.
   In der Regel liegt die Reaktionstemperatur im Bereich von 0°C bis zur Höhe des Siedepunktes des Reaktionsgemisches.
   Die Aufarbeitung kann in an sich bekannter Weise zum Produkt hin erfolgen.
B. Darstellung von Verbindungen der Formel I mit R⁸ = OR¹¹, OPOR¹²R¹³ oder OPSR¹²R¹³ durch Umsetzung von Pyrazolon-Derivaten der Formel III mit Alkylierungs- bzw. Phosphonylierungsmitteln IVα, IVβ bzw. IVγ. 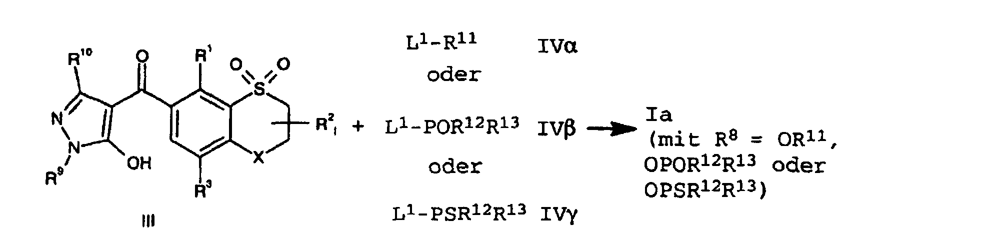
   L¹ steht für eine nucleophil verdrängbare Abgangsgruppe, wie Halogen, z. B. Chlor oder Brom, Hetaryl, z. B. Imidazolyl, Carboxylat, z. B. Acetat, oder Sulfonat, z. B. Mesylat oder Triflat etc.
   Die Verbindungen der Formel IVα, IVβ oder IVγ können direkt eingesetzt werden wie z. B. im Fall der Carbonsäurehalogenide oder in situ erzeugt werden, z. B. aktivierte Carbonsäuren (mit Carbonsäure und Dicyclohexylcarbodiimid etc.).
   Die Ausgangsverbindungen werden in der Regel im äquimolaren Verhältnis eingesetzt. Es kann aber auch von Vorteil sein, die eine oder andere Komponente im Überschuß einzusetzen.
   Gegebenenfalls kann es von Vorteil sein, die Umsetzungen in Gegenwart einer Base durchzuführen. Die Reaktanden und die Base werden dabei zweckmäßigerweise in äquimolaren Mengen eingesetzt. Ein Überschuß der Base z.B. 1,5 bis 3 Moläquivalente kann unter Umstanden vorteilhaft sein.
   Als Basen eignen sich tertiäre Alkylamine, wie Triethylamin, aromatische Amine, wie Pyridin, Alkalimetallcarbonate, z.B. Natriumcarbonat oder Kaliumcarbonat, Alkalimetallhydrogencarbonate, wie Natriumhydrogencarbonat und Kaliumhydrogencarbonat, Alkalimetallalkoholate wie Natriummethanolat, Natriumethanolat, Kalium-tert.-butanolat oder Alkalimetallhydride, z.B. Natriumhydrid. Bevorzugt verwendet werden Triethylamin oder Pyridin.
   Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.
   In der Regel liegt die Reaktionstemperatur in Bereich von 0°C bis zur Höhe des Siedepunktes des Reaktionsgemisches.
   Die Aufarbeitung kann in an sich bekannter Weise zum Produkt hin erfolgen.
C. Darstellung von Verbindungen der Formel I mit R⁸ = OR¹¹, SR¹¹, POR¹²R¹³, NR¹⁴R¹⁵, 0NR¹⁵R¹⁵, N-gebundenes Heterocyclyl oder O-(N-gebundenes Heterocyclyl) durch Umsetzung von Verbindungen der Formel I mit R⁸ = Halogen (Iα) mit Verbindungen der Formel Vα, Vβ, Vγ, Vδ, Vε, Vη oder Vϑ, gegebenenfalls in Gegenwart einer Base oder unter vorangehender Salzbildung. 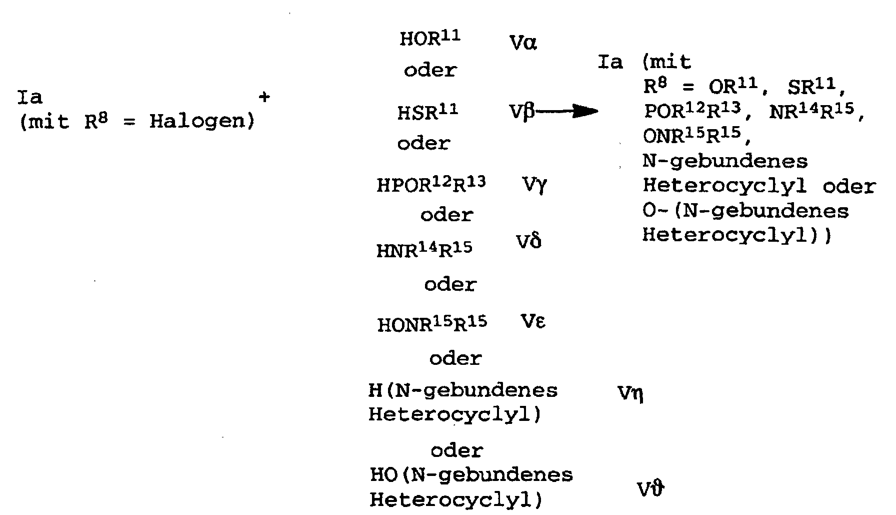
   Die Ausgangsverbindungen werden in der Regel im äquimolaren Verhältnis eingesetzt. Es kann aber auch von Vorteil sein, die eine oder andere Komponente im Überschuß einzusetzen.
   Gegebenenfalls kann es von Vorteil sein, die Umsetzungen in Gegenwart einer Base durchzuführen. Die Reaktanden und die Base werden dabei zweckmäßigerweise in äquimolaren Mengen eingesetzt. Ein Überschuß der Base z.B. 1,5 bis 3 Moläquivalente, bezogen auf Ia und/oder Ib (mit R⁸ = Halogen) oder III, kann unter Umständen vorteilhaft sein.
   Als Basen eignen sich tertiäre Alkylamine, wie Triethylamin, aromatische Amine, wie Pyridin, Alkalimetallcarbonate, z.B. Natriumcarbonat oder Kaliumcarbonat, Alkalimetallhydrogencarbonate, wie Natriumhydrogencarbonat und Kaliumhydrogencarbonat, Alkalimetallalkoholate wie Natriummethanolat, Natriumethanolat, Kalium-tert.-butanolat oder Alkalimetallhydride, z.B. Natriumhydrid. Bevorzugt verwendet werden Natriumhydrid oder Kalium-tert.-butylat.
   Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether,wie Diethylether, Methyl-tert.-butylether, Tetrahydrofuran oder Dioxan, polare aprotische Lösungsmittel, wie Acetonitril, Dimethylformamid oder Dimethylsulfoxid oder Gemische hiervon in Betracht.
   In der Regel liegt die Reaktionstemperatur im Bereich von 0°C bis zur Höhe des Siedepunktes des Reaktionsgemisches.
   Die Aufarbeitung kann in an sich bekannter Weise zum Produkt hin erfolgen.
D. Darstellung von Verbindungen der Formel I mit R⁸ = SOR¹², SO₂R¹² durch Umsetzung von Verbindungen der Formel I mit R⁸ = SR¹² (Iβ) mit einem Oxidationsmittel. 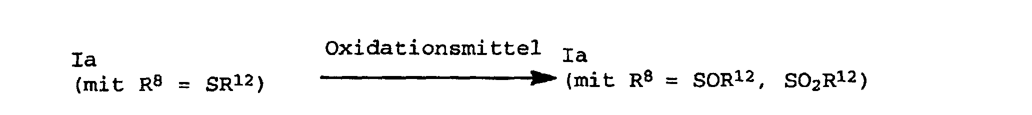
   Als Oxidationsmittel kommen beispielsweise m-Chlorperbenzoesäure, Peroxyessigsäure, Trifluorperoxyessigsäure, Wasserstoffperoxid, ggf. in Gegenwart eines Katalysators wie Wolframat, in Betracht.
   Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.
   In der Regel liegt die Reaktionstemperatur im Bereich von 0°C bis zur Höhe des Siedepunktes des Reaktionsgemisches.
   Die Aufarbeitung kann in an sich bekannter Weise zum Produkt hin erfolgen.
E. Darstellung von Verbindungen der Formel I mit R⁷ = IIa durch Umsetzung eines metallierten Pyrazol-Derivats der Formel VI mit einem Dioxothiochromancarbonsäure-Derivat der Formel VII:

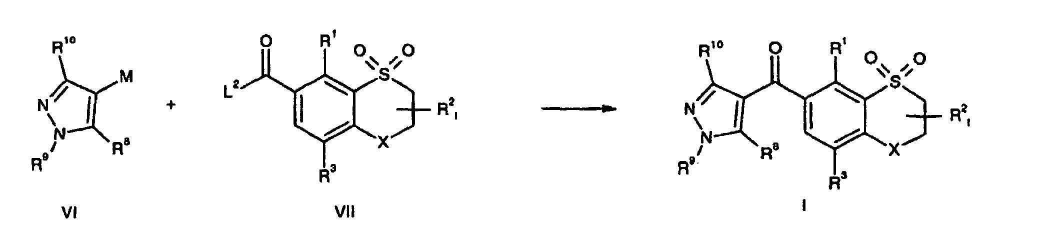

M steht hierbei für ein Metall, insbesondere für ein Alkalimetall wie Lithium oder Natrium, ein Erdalkalimetall wie z.B. Magnesium oder ein Übergangsmetall wie Palladium, Nickel etc. und L² für eine nucleophil verdrängbare Abgangsgruppe wie Halogen, z.B. Chlor oder Brom, Alkylsulfonat wie Mesylat, Halogenalkylsulfonat wie Triflat oder Cyanid.

Die Umsetzung wird in der Regel bei Temperaturen von -100°C bis Rückflußtemperatur des Reaktionsgemisches durchgeführt. Als Lösungsmittel eignen sich inerte aprotische Lösungsmittel, wie Ether, z.B. Diethylether, Tetrahydrofuran. Die Verbindungen der Formel VII werden in der Regel im Überschuß eingesetzt, es kann aber auch von Vorteil sein, diese in äquimolaren Mengen oder im Unterschuß einzusetzen. Die Aufarbeitung erfolgt zum Produkt hin.

In Abhängigkeit von den Reaktionsbedingungen können die Verbindungen Ia, Ib (= Verbindungen der Formel I mit R⁷ = IIb) oder Gemische hiervon gebildet werden. Letztere können durch klassische Trennmethoden, wie z.B. Kristallisation, Chromatographie etc., getrennt werden.

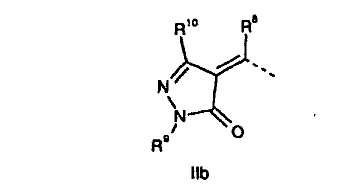

Die Pyrazolon-Derivate der Formel III sind bekannt oder können nach an sich bekannten Verfahren hergestellt werden (z.B. DE 19 532 312). Beispielsweise durch Umsetzung von Pyrazolonen der Formel VIII mit einer aktivierten Benzoesäure VIIa oder einer Benzoesäure VIIb, die vorzugsweise in situ aktiviert wird, zu dem Acylierungsprodukt und anschließende Umlagerung.

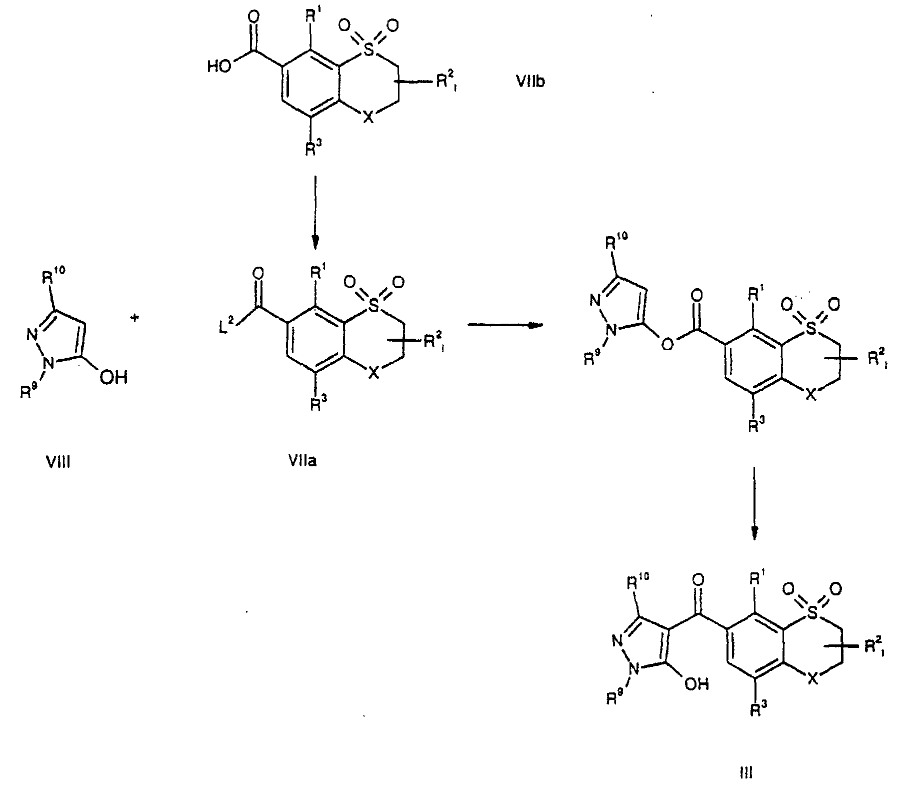

L² steht für eine nucleophil verdrängbare Abgangsgruppe, wie Halogen z.B. Brom oder Chlor, Hetaryl, z.B. Imidazolyl oder Pyridyl, Carboxylat, z.B. Acetat oder Trifluoracetat etc.

Die aktivierte Benzoesäure VIIa kann direkt eingesetzt werden, wie im Fall der Benzoylhalogenide oder in situ erzeugt werden, z.B. mit Dicyclohexylcarbodiimid, Triphenylphosphin/Azodicarbonsäureester. 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf VII, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel III erfolgt zweckmäßigerweise bei Temperaturen von 20 bis 100°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonat verwendet, vorzugsweise in doppelt äquimolaren Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid oder Essigsäureethylester extrahiert. Der organische Extrakt kann mit 5-10%iger Alkalicarbonatlösung, z.B. Natriumcarbonat- oder Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.

Die Benzoylhalogenide der Formel VIIa (mit L² = Cl, Br) können auf an sich bekannte Art und Weise durch Umsetzung der Benzoesäuren der Formel VIIb mit Halogenierungsreagentien wie Thionylchlorid, Thionylbromid, Phosgen, Diphosgen, Triphosgen, Oxalylchlorid, Oxalylbromid hergestellt werden.

Die Benzoesäuren der Formel VIIb können in bekannter weise durch saure oder basische Hydrolyse aus den entsprechenden Estern hergestellt werden.

Die metallierten Pyrazol-Derivate der Formel VI können auf an sich bekannte Art und Weise durch Umsetzung von in 4-Position halogenierten Pyrazolen mit Metallen wie Lithium, Natrium, Magnesium etc. oder mit metallorganischen Verbindungen wie z.B. Butyllithium gebildet werden. Es ist aber auch möglich Pyrazole, die in 4-Position mit Wasserstoff verknüpft sind, direkt zu metallieren, z.B, mit den voranstehend genannten Metallen bzw. metallorganischen Verbindungen. Die Umsetzungen werden in der Regel in einem inerten aprotischen Lösungsmittel durchgeführt, bevorzugt in Ether wie Diethylether, Tetrahydrofuran etc.. Die Reaktionstemperatur liegt im Bereich von -100°C bis zur Höhe des Siedepunktes des Reaktionsgemisches. Die Verbindungen der Formel VI werden in der Regel direkt weiter umgesetzt oder in situ erzeugt.

### Herstellungsbeispiele:

### 5-Chlor-1-ethyl-4-(4,4,8-trimethyl-1,1-dioxothiochroman-7-yl)-carbonyl-pyrazol (Verbindung 2.1)

### Stufe a) 3-(3-Methyl-2-butenylthio)-2-methyl-benzoesäuremethylester

Zu 50 g (0.275 mol) 3-Thio-2-methyl-benzoesäuremethylester in 250 ml Aceton wurden 37.9 g (0.275 mol) Kaliumcarbonat und 43.5 g (0.275 mol) 3-Methyl-2-butenylbromid getropft und zehn Stunden bei Raumtemperatur gerührt. Nach Abdestillieren des Lösungsmittels wurde der Rückstand in Wasser/Essigsäureethylester aufgenommen, die organische Phase getrocknet, abfiltriert und eingeengt. Ausbeute : 67.9 g (98.9%) gelbes Öl.
¹H-NMR (CDCl₃, δ in ppm): 7.63 (d,1H); 7.41 (d,1H); 7.16 (t,1H);
5.25 (m,1H); 3.90 (s,3H),3.49 (d,2H);
2.60 (s,3H); 1.70 (s,3H); 1.56 (s,3H).

### Stufe b) 4,4,8-Trimethylthiochroman-7-carbonsäuremethylester

67.9 g (0.27 mol) 3-(3-Methyl-2-butenylthio)-2-methylbenzoesäuremethylester wurden in 600 ml Methylenchlorid gelöst, 206,4 g (1,09 mol) Titantetrachlorid in 600 ml Methylenchlorid bei -5 bis 0°C zugetropft und drei Stunden bei 0°C nachgerührt. Anschließend wurde die Mischung in 1.5 kg Eis und 500 ml gesättigte Ammoniumchlorid-Lösung eingerührt, die organische Phase abgetrennt, getrocknet und das Lösungsmittel entfernt. Man erhielt 62.9 g eines orangen Öls, das direkt weiter eingesetzt wurde. Zur Produktcharakterisierung wurde eine Probe an Kieselgel (Eluent: Cyclohexan/ Essigsäureethylester = 10/1) chromatographiert.
Schmelzpunkt: 63°C

### Stufe c) 4,4,8-Trimethylthiochroman-7-carbonsäure

62.9 g 4,4,8-Trimethylthiochroman-7-carbonsäuremethylester wurden in 600 ml eines 1 : 1 Wasser/Methanol-Gemisches vorgelegt und 15.1 g (0.377 mol) Natriumhydroxid zugegeben. Die Lösung wurde dann drei Stunden unter Rückfluß erhitzt, das organische Lösungsmittel entfernt, 200 ml Wasser zugegeben und unter Kühlung mit konz. Salzsäure sauer gestellt. Der ausgefallene Niederschlag wurde abgesaugt, mit Wasser gewaschen und getrocknet.
Ausbeute: 57.2 g
Schmelzpunkt: 212°C

### Stufe d) 4,4,8-Trimethyl-1,1-dioxothiochroman-7-carbonsäure

57.2 g (0.24 mol) 4,4,8-Trimethylthiochroman-7-carbonsäure wurden in 500 ml Essigsäure gelöst und eine Sgatelspitze Natriumwolframat zugegeben. Bei 50 bis 60°C wurden 60.4 g (0.53 mol) 30 %iges Wasserstoffperoxid zugetropft, drei Stunden bei 50°C nachgerührt, in Eiswasser eingerührt und die ausgefallenen weißen Nadeln abfiltriert, mit Wasser gewaschen und getrocknet.
Ausbeute: 47.2 g (72.7 %)
Schmelzpunkt: 280°C (Zersetzung)

### Stufe e) 4,4,8-Trimethyl-1,1-dioxothiochroman-7-carbonsäurechlorid

20.0 g (0.075 mol) 4,4,8-Trimethyl-1,1-dioxothiochroman-7-carbonsäure wurden in 200 ml Toluol gelöst, drei Tropfen Dimethylformamid sowie 10.7 g (0.09 mol) Thionylchlorid zugegeben. Nach drei Stunden Erhitzen unter Rückfluß wurde das Lösungsmittel entfernt und das zurückbleibende farblose Öl (Ausbeute 21.3 g) direkt weiter eingesetzt.

### Stufe f) 5-Chlor-1-ethyl-4-(4,4,8-trimethyl-1,1-dioxothiochroman-7-yl)-carbonyl-pyrazol (Verbindung 2.1)

Zu 0.86 g (4.11 mmol) 4-Brom-5-Chlor-1-ethyl-pyrazol in 30 ml Diethylether wurden unter Schutzgasatmosphäre bei 20 bis 25 °C schnell 2.6 ml (4.11 mmol) n-Buthyllithium in Hexan (1.6 molar) getropft. Nach 15 Minuten Rühren bei Raumtemperatur wurde die resultierende Suspension bei -70°C zu einer Lösung von 2.2 g (8.21 mmol) 4,4,8-Trimethyl-1,1-dioxothiochroman-7-carbonsäurechlorid in 30 ml Diethylether getropft. Anschließend wurde der Reaktionsansatz auf Raumtemperatur erwärmt und mit 11 ml Methanol und 26 ml 2N Natronlauge versetzt. Die organische Phase wurde dann abgetrennt, getrocknet, eingeengt und der Rückstand an kieselgel (Eluent: Essigsäureethylester/Cyclohexan = 3/2) chromatographiert.
Ausbeute: 0.17 g
Schmelzpunkt: 131-133 °C

### 1-Ethyl-4-(8-methyl-2,3-dihydro-1,1,4,4-tetraoxobenz [1,4] dithiin-7-ylcarbonyl)-5-propylcarbonyloxy-pyrazol (Verbindung 2.5)

### Stufe a) 3- (2-Bromethylthio)-2-methyl-benzoesäuremethylester

Zu 40.0 g (0.22 mol) 3-Thio-2-methyl-benzoesäuremethylester in 500 ml Aceton wurden 30.3 g (0.22 mol) Kaliumcarbonat gegeben und 82.6 g (0.22 mol) 1,2-Dibromethan getropft. Nach zehn Stunden Rühren bei Raumtemperatur wurde das Lösungsmittel abdestilliert, der Rückstand in Wasser/Essigsäureethylester aufgenommen, die organische Phase getrocknet und eingeengt. Das zurückbleibende Öl wurde mit Essigsäureester/Cyclohexan = 1/10 über Kieselgel chromatographiert.
Ausbeute : 42.7 g (67.2 %) farblose Kristalle.
¹H-NMR (CDCl₃, δ in ppm): 7.68 (d,1H); 7.51 (d,1H); 7.20 (t, 1H);
3.90 (s, 3H), 3.41 (m, 2H); 3.25 (m, 2H);
2.62 (s, 3H).

### Stufe b) 3-(2-Methylsulfonylthioethylthio)-2-methyl-benzoesäuremethylester

Zu 42.7 g (0.148 mol) 3-(2-Bromethylthio)-2-methyl-benzoesäuremethylester in 400 ml Ethanol wurden 33.2 g (0.22 mol) Kalium-Methylsulfonylthiolat gegeben und fünf Stunden unter Rückfluß erhitzt. Nach Entfernen des Lösungsmittel wurde der Rückstand in Wasser/Essigsäureethylester aufgenommen, getrocknet und eingeengt. Das zurückbleibende Öl wurde mit Essigsäureethylester/Cyclohexan = 1/4 an Kieselgel chromatographiert.
Ausbeute : 33.2 g (67.2 %) gelbes Öl.
Schmelzpunkt: 55°C

### Stufe c) 8-Methyl-2,3-dihydro-benz[1,4]dithiin -7-carbonsäuremethylester

49.2 g (0.154 mol) 3-(2-Methylsulfonylthioethylthio)-2-methylbenzoesäuremethylester wurden in 500 ml Methylenchlorid gelöst, 80.2 g (0.308 mol) Zinntetrachlorid zugegeben, drei Stunden unter Rückfluß erhitzt und zehn Stunden bei Raumtemperatur nachgerührt. Die Mischung wurde dann mit Wasser und gesättigter Natriumhydrogencarbonatlösung gewaschen, die organische Phase abgetrennt, getrocknet und eingeengt. Das zurückbleibende Öl wurde mit Essigsäureethylester/Cyclohexan = 1/10 an Kieselgel chromatographiert.
Ausbeute : 17.1 g (46.3 %)farbloses Öl
Schmelzpunkt: 57°C

### Stufe d) 8-Methyl-2,3-dihydro-benz[1,4]dithiin -7-carbonsäure

9.6 g (0.04 mol) 8-Methyl-2,3-dihydro-benz[1,4]dithiin-7-carbonsäuremethylester wurden in 100 ml eines 1 : 1 Gemisches aus Wasser/Methanol vorgelegt und 2.4 g (0.06 mol) Natriumhydroxid zugegeben. Die Lösung wurde zwei Stunden unter Rückfluß erhitzt, dann das organische Lösungsmittel abdestilliert, 200 ml Wasser zugegeben und unter Kühlung mit konz. Salzsäure sauer gestellt. Der ausgefallene Niederschlag wird abgesaugt, mit Wasser gewaschen und getrocknet.
Ausbeute: 8.1 g (89,6 %) farblose Kristalle
Schmelzpunkt: 175°C

### Stufe e) 8-Methyl-2,3-dihydro-1,1,4,4-tetraoxobenz[1,4]dithiin-7-carbonsäure

19.4 g (0.086 mol) 8-Methyl-2,3-dihydro-benz[1,4]dithiin-7-carbonsäure wurden in 200 ml Essigsäure gelöst und eine Spatelspitze Natriumwolframat zugegeben. Bei 50 - 60°C wurden dann hierzu 42.8 g (0.38 mol) 30 %iges Wasserstoffperoxid getropft. Nach fünf Stunden Rühren bei 50°C wurde abgekühlt, in Eiswasser eingerührt und die ausgefallenen weißen Nadeln abfiltriert. mit Wasser gewaschen und getrocknet.
Ausbeute: 21.7 g (87.2 %)
Schmelzpunkt: 282°C

### Stufe f) 8-Methyl-2,3-dihydro-1,1,4,4-tetraoxobenz[1,4]dithiin-7-carbonsäurechlorid

10.0 g (0.0345 mol) 8-Methyl-2,3-dihydro-1,1,4,4-tetraoxobenz [1,4] dithiin-7-carbonsäure wurden in 100 ml Toluol gelöst. zwei Tropfen Dimethylformamid und anschließend 4.5 g (0.038 mol) Thionylchlorid zugegeben. Nach vier Stunden Rühren unter Rückfluß wurde eingeengt. Das zurückbleibende farblose Öl (Ausbeute 10.6 g) konnte direkt weiter eingesetzt werden.

### Stufe g) 1-Ethyl-5-hydroxy-4-(8-methyl-2,3-dihydro-1,1,4,4-tetraoxobenz[1,4]dithiin-7-yl)-carbonyl-pyrazol

5.0 g (0.017 mol) 8-Methyl-2,3-dihydro-1,1,4,4-tetraoxobenz[1,4]dithiin-7-carbonsäure wurden in 50 ml Acetonitril gelöst, 1.93 g (0.017 mol) 1-Ethyl-5-hydroxypyrazol und 3.56 g (0.017 mol) N,N-Dicyclohexylcarbodiimid zugegeben. Nach einer Stunde Rühren bei 20°C wurde der Reaktionsansatz dann in 100 ml 2%-ige Natriumhydrogencarbonat-Lösung aufgenommen, der sich bildende Niederschlag abgesaugt, das Filtrat getrocknet und eingeengt. Anschließend wurde der zurückbleibende weiße Feststoff (5.8 g) in 15 ml Dioxan gelöst, 4.2 g ( 0.03 mol) Kaliumcarbonat zugegeben und 3 Stunden unter Rückfluß erhitzt. Nach Abkühlen wurde Wasser zugegeben, mit Essigsäureethylester gewaschen, die wäßrige Phase mit 2N Salzsäure auf pH 3 gestellt, der ausfallende Niederschlag abgesaugt, mit Wasser gewaschen und getrocknet. Ausbeute: 2.6 g (37.8 %) farblose Kristalle.
1H-NMR (CDCl₃, δ in ppm): 7.97 (d, 1H); 7.80 (d, 1H);
7.45 (s, 1H); 9.41 (s, 4H); 3.90 (q,2H);
2.59 (s, 3H); 1.26 (t, 3H).

### Stufe h) 1-Ethyl-4-(8-methyl-2,3-dihydro-1,1,4,4-tetraoxobenz[1,4]dithiin-7-yl)-carbonyl-5-propylcarbonyloxypyrazol

1.5 g (3.9 mmol) 1-Ethyl-5-hydroxy-4-(8-methyl-2,3-dihydro-1,1,4,4-tetraoxobenz[1,4]dithiin-7-yl)-carbonyl-pyrazol wurden in 20 ml Methylenchlorid suspendiert, 0.42 g (3.9 mmo1) Buttersäurechlorid und 0.39 g (0.42 mol) Triethylamin zugegeben. Nach fünf Stunden Rühren bei 20°C wurde mit 2N Salzsäure , Natriumhydrogencarbonat-Lösung und Wasser gewaschen, getrocknet, abfiltriert und das Lösungsmittel abdestilliert. Der Rückstand wurde anschließend an Kieselgel (Eluent: Essigsäureethylester/Cyclohexan = 2/3) chromatographiert.
Ausbeute: 0.85 g (50 %) farblose Kristalle.
Schmelzpunkt: 90°C

In Tabelle 2 sind neben den voranstehenden Verbindungen noch weitere pyrazolyldioxothiochromanoyl-Derivate der Formel I aufgeführt, die in analoger Weise hergestellt wurden oder herstellbar sind:

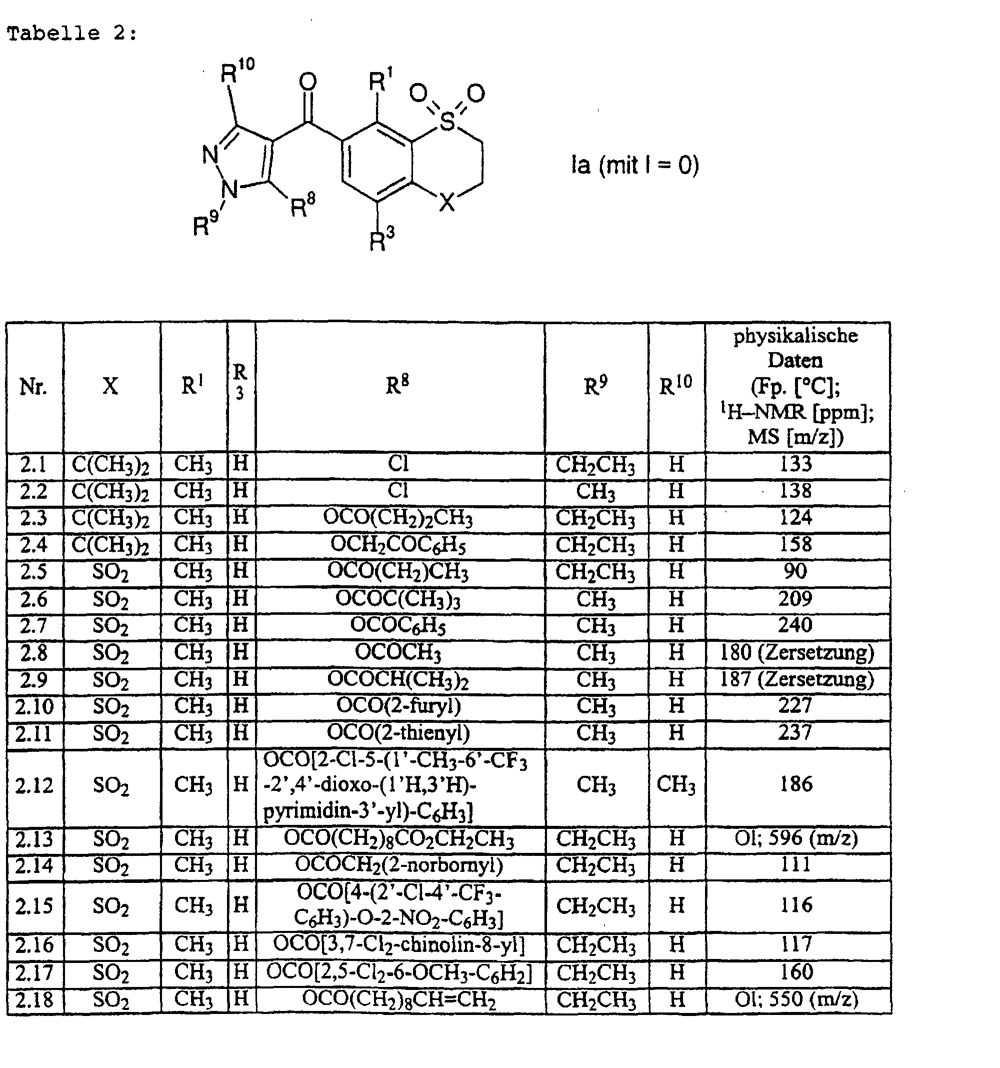

Die Verbindungen der Formel I und deren landwirtschaftlich brauchbaren Salze eignen sich sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline und deren Derivate, alkylierte Benzole oder deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Pyrazolyldioxothiochromanoyl-Derivate der Formel I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.4 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 2.5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile der Verbindung Nr. 2.6 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile der Verbindung Nr. 2.8 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile der Verbindung Nr. 2.10 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile der Verbindung Nr. 2.9 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung Nr. 2.11 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung Nr. 2.12 wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger vertraglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

Zur Verbreiterung des Wirkungsspektrums und zur Erzielung synergistischer Effekte können die Pyrazolyldioxothiochromanoyl-Derivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-Aroyl-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessiqi säure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder 5 phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der Pyrazolyldioxothiochromanoyl-Derivate der Formel I ließ sich durch die folgenden Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgzerten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufbehandlung betrug 0.25 bzw. 0.125 kg/ha a.S.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0.225 bzw. 0.0625 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Chenopodium album | Weißer Gänsefuß | lambsquarters (goosefoot) |
| Echinochloa crus-galli | Hühnerhirse | barnyardgrass |
| Galium aparine | Klettenlabkraut | catchweed bedstraw |
| Ipomoea spp. | Prunkwindenarten | morningglory |
| Polygonum persicaria | Flohknöterich | ladysthumb |
| Setaria faberi | Borstenhirse | giant foxtail |
| Setaria viridis | Grüne Borstenhirse | green foxtail |
| Sinapis alba | Weißer Senf | white mustard |
| Solanum nigrum | Schwarzer Nachtschatten | black nightshade |
| Zea mays | Mais | corn |

Bei Aufwandmengen von 0.125 bzw. 0.0625 kg/ha zeigt die Verbindung 2.8 im Nachauflauf eine sehr gute Wirkung gegen die unerwünschten Pflanzen weißer Gänsefuß, Flöhknöterich, Borstenhirse, grüne Borstenhirse sowie weißer Senf. Ebenso zeigte die Verbindung 2.12 bei voranstehend genannten Aufwandmengen im Nachauflauf eine sehr gute Wirkung gegen weißen Gänsefuß, Hühnerhirse, Klettenlabkraut, Prunkwindenarten und schwarzen Nachtschatten. Weiterhin bekämpfte Verbindung 2.5 im Vorauflauf bei Aufwandmengen von 0.25 bzw. 0.125 kg/ha unerwünschte Gräser wie Hühnerhirse und Borstenhirse sehr gut unter gleichzeitiger Maisverträglichkeit.

## Patentansprüche

1. Pyrazolyldioxothiochromanoyl-Derivate der Formel I 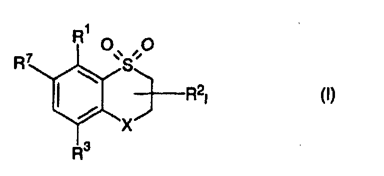 in der die Variablen folgende Bedeutungen haben:
X S(=O)₂ oder CR⁴R⁵;
R¹ Wasserstoff, Nitro, Halogen, Cyano, C₁-C₆-Alkyl, C₁-C₆-Halogenalkyl, C₂-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Aminosulfonyl, N-(C₁-C₆-Alkyl)-aminosulfonyl, N,N-Di-(C₁-C₆-alkyl)-aminosulfonyl, N-(C₁-C₆-Alkylsulfonyl)-amino, N-(C₁-C₆-Halogenalkylsulfonyl)-amino, N-(C₁-C₆-Alkyl)-N-(C₁-C₆-alkylsulfonyl)-amino oder N-(C₁-C₆-Alky)-N-(C₁-C₆-halogenalkylsulforiyl)-amino;
R² C₁-C₆-Alkyl, C₁-C₆-Halogenalkyl, C₁-C₆-Alkoxy oder C₁-C₆-Halogenalkoxy;
R³ Wasserstoff, C₁-C₆-Alkyl oder Halogen;
R⁴,R⁵ Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, N-C₁-C₆-Alkylamino, N-C₁-C₆-Halogenalkylamino, N,N-Di-(C₁-C₆-alkyl)-amino, N-C₁-C₆-Alkoxyamino, N-(C₁-C₆-Alkoxy)-N-(C₁-C₆-alkyl) amino, 1-Tetrahydropyrrolyl, 1-Piperidinyl, 4-Morpholinyl oder 1-Hexahydropyrazinyl;
oder
R⁴ und R⁵ bilden gemeinsam eine -O-(CH₂)ₘ-O-, -O-(CH₂)ₘ-S-, - S-(CH₂)ₘ-S- oder -O-(CH₂)ₙ-Kette, die durch einen bis drei Reste aus folgender Gruppe substituiert sein kann:
Halogen, Cyano, C₁-C₄-Alkyl, C₁-C₄-Halogenalkyl oder C₁-C₄-Alkoxycarbonyl;
oder
R⁴ und R⁵ bilden gemeinsam eine -(CH₂)ₚ-Kette, die durch Sauerstoff oder Schwefel unterbrochen sein kann und/oder durch einen bis vier Reste aus folgender Gruppe substituiert sein kann:
Halogen, Cyano, C₁-C₄-Alkyl, C₁-C₄-Halogenalkyl oder C₁-C₄-Alkoxycarbonyl;
oder
R⁴ und R⁵ bilden gemeinsam eine Methylidengruppe, die durch einen bis zwei Reste aus folgender Gruppe substituiert sein kann:
Halogen, Cyano, Hydroxy, Formyl, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
l 0 bis 4;
m 2 bis 4;
n 1 bis 5;
p 2 bis 5;
R⁷ eine Verbindung IIa 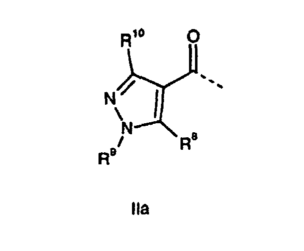
wobei
R⁸ Halogen, OR¹¹, SR¹¹, SOR¹², SO₂R¹², POR¹²R¹³, OPOR¹²R¹³, OPSR¹²R¹³, NR¹⁴R¹⁵, ONR¹⁵R¹⁵, N-gebundenes Heterocyclyl oder O-(N-gebundenes Heterocyclyl), wobei der Heterocyclyl-Rest der beiden letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R⁹ Wasserstoff, C₁-C₆-Alkyl, C₁-C₆-Halogenalkyl, Hydroxy, C₁-C₆-Alkoxy oder C₁-C₆-Halogenalkoxy;
R¹⁰ Wasserstoff, Halogen, C₁-C₆-Alkyl, C₁-C₆-Halogenalkyl, Hydroxy, C₁-C₆-Alkoxy, C₁-C₆-Halogenalkoxy, C₁-C₆-Alkylthio oder C₁-C₆-Halogenalkylthio;
R¹¹ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, C₁-C₂₀-Alkylcarbonyl, C₂-C₂₀-Alkenylcarbonyl, C₂-C₆-Alkinylcarbonyl, C₃-C₆-Cycloalkylcarbonyl, (2-Norbornyl)methylcarbonyl; C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl oder N,N-Di-(C₁-C₆-alkylamino)-imino-C₁-C₆-alkyl, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenylcarbonyl-C₁-C₆-alkyll Heterocyclylcarbonyl-C₁-C₆-alkyl, Phenylcarbonyl, Heterocyclylcarbonyl, Phenoxycarbonyl, Phenyloxythiocarbonyl, Heterocyclyloxycarbonyl, Heterocyclyloxythiocarbonyl, Phenylaminocarbonyl, N-(C₁-C₆Alkyl)-N-(phenyl)-aminocarbonyl, Heterocyclylaminocarbonyl, N-(C₁-C₆-Alkyl)-N-(heterocyclyl)-aminocarbonyl, Phenyl-C₂-C₆-alkenylcarbonyl oder Heterocyclyl-C₂-C₆-alkenylcarbonyl, wobei der Phenyl- und der Heterocyclyl-Rest der 18 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy, C₁-C₄-Halogenalkoxy, Phenoxy, Heterocyclyl oder N-gebundenes Heterocyclyl, wobei die drei letztgenannten Substituenten ihrerseits partiell oder vollständig halogeniert sein können und/oder einen bis drei der folgenden Reste tragen können: Nitro, Cyano, C₁-C₄-Alkyl, C₁-C₄-Halogenalkyl, C₁-C₄-Alkoxy oder C₁-C₄-Halogenalkoxy;
R¹², R¹³ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cyclo- alkyl, Hydroxy, C₁-C₆-Alkoxy, Amino, C₁-C₆-Alkylamino, C₁-C₆-Halogenalkylamino, Di-(C₁-C₆-alkyl)amino oder Di-(C₁-C₆-Halogenalkyl)amino, wobei die genannten Alkyl-, Cycloalkyl- und Alkoxyreste partiell oder vollständig halogeniert sein können und/oder eine bis drei der folgenden Gruppen tragen können:
Cyano, C₁-C₄-Alkoxy, C₁-C₄-Alkylthio, Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl, Heterocyclyl-C₁-C₆-alkyl, Phenoxy, Heterocyclyloxy, wobei der Phenyl- und der Heterocyclyl-Rest der letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R¹⁴ C₁-C₆-Alkyl, C₃-C₆-Alkenyl, C₃-C₆-Halogenalkenyl, C₃-C₆-Alkinyl, C₃-C₆-Halogenalkinyl, C₃-C₆-Cycloalkyl, Hydroxy, C₁-C₆-Alkoxy, C₃-C₆-Alkenyloxy, C₃-C₆-Alkinyloxy, Amino, C₁-C₆-Alkylamino, Di-(C₁-C₆-alkyl)-amino oder C₁-C₆-Alkylcarbonylamino, wobei die genannten Alkyl-, Cycloalkyl- und Alkoxyreste partiell oder vollständig halogeniert sein können und/oder einen bis drei Reste der folgenden Gruppe tragen können:
Cyano, C₁-C₄-Alkoxy, C₁-C₄-Alkylthio,
Di-(C₁-C₄-alkyl)-amino, C₁-C₄-Alkylcarbonyl, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, Hydroxycarbonyl, C₁-C₄-Alkylaminocarbonyl, Di-(C₁-C₄-alkyl)-aminocarbonyl, Aminocarbonyl, C₁-C₄-Alkylcarbonyloxy oder C₃-C₆-Cycloalkyl;
Phenyl, Heterocyclyl, Phenyl-C₁-C₆-alkyl oder Heterocyclyl-C₁-C₆-alkyl, wobei der Phenyl- oder Heterocyclyl-Rest der vier letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R¹⁵ C₁-C₆-Alkyl, C₃-C₆-Alkenyl, C₃-C₆-Alkinyl oder C₁-C₆-Alkylcarbonyl;
sowie deren landwirtschaftlich brauchbaren Salze.

2. Verfahren zur Herstellung von Verbindungen der Formel I gemäß Anspruch 1 mit R⁸ = Halogen, **dadurch gekennzeichnet, daß** man ein Pyrazolon-Derivat der Formel III, 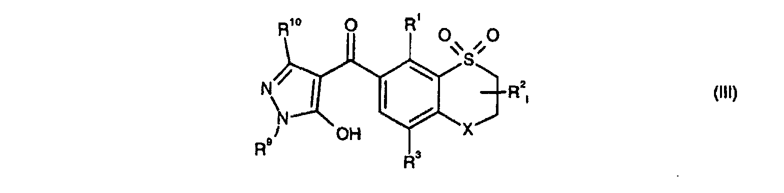 wobei die Variablen R¹ bis R¹⁰, X, l die in Anspruch 1 genannte Bedeutung haben, mit einem Halogenierungsmittel umsetzt.

3. Verfahren zur Herstellung von Verbindungen der Formel I gemäß Anspruch 1 mit R⁸ = OR¹¹, OPOR¹²R²³ oder OPSR¹²R¹³ **dadurch gekennzeichnet, daß** man ein Pyrazolon-Derivat der Formel III, 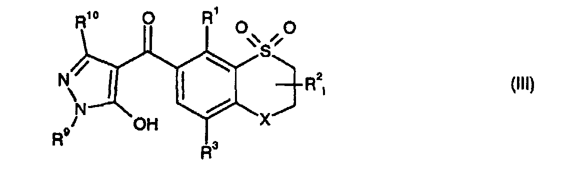 wobei die Variablen R¹ bis R¹⁰, X, l die in Anspruch 1 genannte Bedeutung haben, mit einer Verbindung der Formel IVα, IVβ oder IVγ, 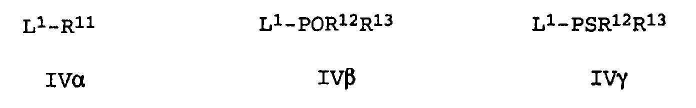 wobei die Variablen R¹¹ bis R¹³ die in Anspruch 1 genannte Bedeutung haben und L¹ für eine nucleophil verdrängbare Abgangsgruppe steht, umsetzt.

4. Verfahren zur Herstellung von Verbindungen der Formel I gemäß Anspruch 1 mit R⁸ = OR¹¹, SR¹¹, POR¹²R¹³, NR¹⁴R¹⁵, ONR¹⁵R¹⁵, N-gebundenes Heterocyclyl oder O(N-gebundenes Heterocyclyl), **dadurch gekennzeichnet, daß** man eine Verbindung der Formel Ia (≡ I mit R⁸ = Halogen), 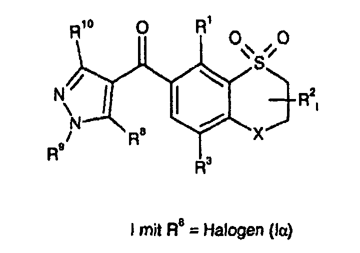 wobei die Variablen R¹ bis R³, R⁹ und R¹⁰, X, l die in Anspruch 1 genannte Bedeutung haben, mit einer Verbindung der Formel Vα, Vβ, Vγ, Vδ, Vε, Vη oder Vϑ, 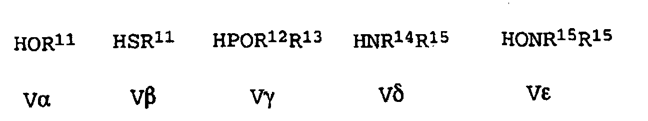 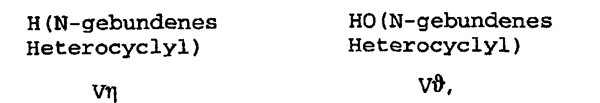 wobei die Variablen R¹¹ bis R¹⁵ die in Anspruch 1 genannte Bedeutung haben, gegebenenfalls in Gegenwart einer Base umsetzt.

5. Verfahren zur Herstellung von Verbindungen der Formel I gemäß Anspruch 1 mit R⁸ = SOR¹², SO₂R¹², **dadurch gekennzeichnet, daß** man eine Verbindung der Formel Iβ (= I mit R⁸ = SR¹²) 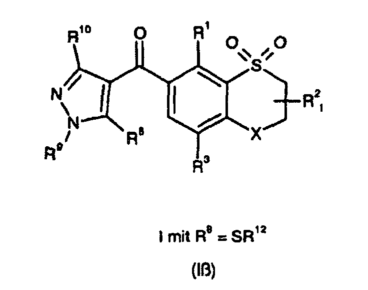 wobei die Variablen R¹ bis R³, R⁹, R¹⁰, X, l die in Anspruch 1 genannte Bedeutung haben, mit einem Oxidationsmittel umsetzt.

6. Verfahren zur Herstellung von Verbindungen der Formel I mit R⁷ = IIa gemäß Anspruch 1, **dadurch gekennzeichnet, daß** man ein metalliertes Pyrazol-Derivat der Formel VI, wobei M für ein Metall steht und R⁸ bis R¹⁰ die in Anspruch 1 genannte Bedeutung haben, mit einem Dioxothiochromancarbonsäure-Derivat der Formel VII, wobei R¹ bis R³, X und l die in Anspruch 1 genannte Bedeutung haben und L² für eine nucleophil verdrängbare Abgangsgruppe steht, umsetzt.

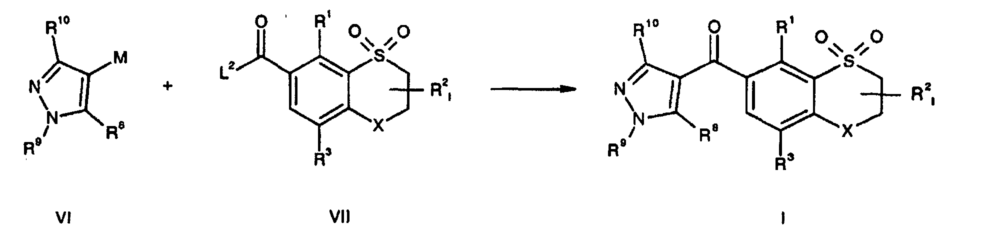

7. Mittel, enthaltend eine herbizid wirksame Menge mindestens eines Pyrazolyldioxothiochromanoyl-Derivates der Formel I oder eines landwirtschaftlich brauchbaren Salzes von I gemäß Anspruch 1, und für die Formulierung von Pflanzenschutzmitteln übliche Hilfsmittel.

8. Verfahren zur Herstellung von Mitteln gemäß Anspruch 7, **dadurch gekennzeichnet, daß** man eine herbizid wirksame Menge mindestens eines Pyrazolyldioxothiochromanoyl-Derivates der Formel I oder eines landwirtschaftlich brauchbaren Salzes von I gemäß Anspruch 1 und für die Formulierung von Pflanzenschutzmitteln übliche Hilfsmittel mischt.

9. Verfahren zur Bekämpfung von unerwünschtem Pflanzenwuchs, **dadurch gekennzeichnet, daß** man eine herbizid wirksame Menge mindestens eines Pyrazolyldioxothiochromanoyl-Derivates der Formel I oder eines landwirtschaftlich brauchbaren Salzes von I gemäß Anspruch 1, auf Pflanzen, deren Lebensraum und/oder auf Samen einwirken läßt.

10. Verwendung von Pyrazolyldioxothiochromanoyl-Derivaten der Formel I oder deren landwirtschaftlich brauchbaren Salze gemäß Anspruch 1 als Herbizide.

## Claims

1. A pyrazolyldioxothiochromanoyl derivative of the formula I 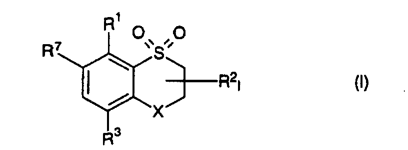 where:
X is S(=O)₂ or CR⁴R⁵;
R¹ is hydrogen, nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, aminosulfonyl, N-(C₁-C₆-alkyl)aminosulfonyl, N,N-di(C₁-C₆-alkyl)aminosulfonyl, N-(C₁-C₆-alkylsulfonyl)amino, N-(C₁-C₆-haloalkylsulfonyl)amino, N-(C₁-C₆-alkyl)-N-(C₁-C₆-alkylsulfonyl)amino or N-(C₁-C₆-alkyl)-N-(C₁-C₆-haloalkylsulfonyl)amino;
R² is C₁-C₆-alkyl, C₁-C₆-haloalkyl, C₁-C₆-alkoxy or C₁-C₆-haloalkoxy;
R³ is hydrogen, C₁-C₆-alkyl or halogen;
R⁴,R⁵ are hydrogen, nitro, halogen, cyano, C₁-C₆-alkyl, C₁-C₆-haloalkyl, C₁-C₆-alkoxy, C₁-C₆-halbalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, N-C₁-C₆-alkylamino, N-C₁-C₆-haloalkylamino, N,N-di(C₁-C₆-alkyl)amino, N-C₁-C₆-alkoxyamino, N-(C₁-C₆-alkoxy)-N-(C₁-C₆-alkyl)amino, 1-tetrahydropyrrolyl, 1-piperidinyl, 4-morpholinyl or 1-hexahydropyrazinyl;
or
R⁴ and R⁵ together form an -O-(CH₂)ₘ-O-, -O-(CH₂)ₘ-S-, -S-(CH₂)ₘ-S- or -O-(CH₂)ₙ- chain which may be substituted by one to three radicals selected from the following group:
halogen, cyano, C₁-C₄-alkyl, C₁-C₄-haloalkyl or C₁-C₄-alkoxycarbonyl;
or
R⁴ and R⁵ together form a -(CH₂)ₚ- chain which may be interrupted by oxygen or sulfur and/or may be substituted by one to four radicals selected from the following group:
halogen, cyano, C₁-C₄-alkyl, C₁-C₄-haloalkyl or C₁-C₄-alkoxycarbonyl;
or
R⁴ and R⁵ together form a methylidene group which may be substituted by one or two radicals selected from the following group:
halogen, cyano, hydroxyl, formyl, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl;
l is 0 to 4;
m is 2 to 4;
n is 1 to 5;
p is 2 to 5;
R⁷ is a compound IIa 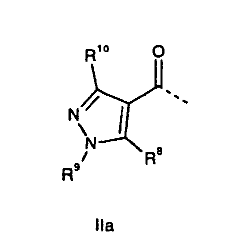
where
R⁸ is halogen, OR¹¹, SR¹¹, SOR¹², SO₂R¹², POR¹²R¹³, OPOR¹²R¹³, OPSR¹²R¹³, NR¹⁴R¹⁵, ONR¹⁵R¹⁵, N-bonded heterocyclyl or O-(N-bonded heterocyclyl), where the heterocyclyl radical of the two lastmentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R⁹ is hydrogen, C₁-C₆-alkyl, C₁-C₆-haloalkyl, hydroxyl, C₁-C₆-alkoxy or C₁-C₆-haloalkoxy;
R¹⁰ is hydrogen, halogen, C₁-C₆-alkyl, C₁-C₆-haloalkyl, hydroxyl, C₁-C₆-alkoxy, C₁-C₆-haloalkoxy,
C₁-C₆-alkylthio or C₁-C₆-haloalkylthio;
R¹¹ is C₁-C₆-alkyl, C₃-C₆-alkenyl, C₃-C₆-haloalkenyl, C₃-C₆-alkynyl, C₃-C₆-haloalkynyl, C₃-C₆-cycloalkyl, C₁-C₂₀-alkylcarbonyl, C₂-C₂₀-alkenylcarbonyl, C₂-C₆-alkynylcarbonyl, C₃-C₆cycloalkylcarbonyl, (2-norbornyl)methylcarbonyl, C₁-C₆-alkoxycarbonyl, C₃-C₆-alkenyloxycarbonyl, C₃-C₆-alkynyloxycarbonyl, C₁-C₆-alkylthiocarbonyl, C₁-C₆-alkylaminocarbonyl, C₃-C₆-alkenylamino- carbonyl, C₃-C₆-alkynylaminocarbonyl, N,N-di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)aminocarbonyl, di(C₁-C₆-alkyl)aminothiocarbonyl, C₁-C₆-alkylcarbonyl-C₁-C₆-alkyl, C₁-C₆-alkoxyimino-C₁-C₆-alkyl, N-(C₁-C₆-alkylamino)imino-C₁-C₆-alkyl or N,N-di(C₁-C₆-alkylamino)imino-C₁-C₆-alkyl, where the abovementioned alkyl, cycloalkyl and alkoxy radicals may be partially or fully halogenated and/or may carry one to three of the following groups:
cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, di(C₁-C₄-alkyl)amino-C₁-C₄-alkoxycarbonyl, hydroxycarbonyl, C₁-C₄-alkylaminocarbonyl, di(C₁-C₄-alkyl)-aminocarbonyl, aminocarbonyl, C₁-C₄-alkyl-carbonyloxy or C₃-C₆-cycloalkyl;
is phenyl, heterocyclyl, phenyl-C₁-C₆-alkyl, heterocyclyl-C₁-C₆-alkyl, phenylcarbonylC₁-C₆-alkyl, heterocyclylcarbonyl-C₁-C₆-alkyl, phenylcarbonyl, heterocyclylcarbonyl, phenoxycarbonyl, phenyloxythiocarbonyl, heterocyclyloxycarbonyl, heterocyclyloxythiocarbonyl, phenylaminocarbonyl, N-(C₁-C₆-alkyl)-N-(phenyl)aminocarbonyl, heterocyclylaminocarbonyl, N-(C₁-C₆-alkyl)-N-(heterocyclyl)aminocarbonyl, phenyl-C₂-C₆-alkenylcarbonyl or heterocyclyl-C₂-C₆-alkenylcarbonyl, where the phenyl and the heterocyclyl radical of the 18 lastmentioned substituents may be partially or fully halogenated and/or may carry one to three of the following radicals:
nitro, cyano, C₁-C₄-alkyl, C₁-C₄-haloalkyl, C₁-C₄-alkoxy, C₁-C₄-haloalkoxy, phenoxy, heterocyclyl or N-bonded heterocyclyl, where the three lastmentioned substituents for their part may be partially or fully halogenated and/or may carry one to three of the following radicals: nitro, cyano, C₁-C₄-alkyl, C₁-C₄-haloalkyl, C₁-C₄-alkoxy or C₁-C₄-haloalkoxy;
R¹², R¹³ are C₁-C₆-alkyl, C₃-C₆-alkenyl, C₃-C₆-haloalkenyl, C₃-₆-alkynyl, C₃-C₆-haloalkynyl, C₃-C₆-cycloalkyl, hydroxyl, C₁-C₆-alkoxy, amino, C₁-C₆-alkylamino, C₁-C₆-haloalkylamino, di(C₁-C₆-alkyl)amino or di(C₁-C₆-haloalkyl)amino, where the abovementioned alkyl, cycloalkyl and alkoxy radicals may be partially or fully halogenated and/or may carry one to three of the following groups:
cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl,
di(C₁-C₄-alkyl)amino-C₁-C₄-alkoxycarbonyl, hydroxycarbonyl, C₁-C₄-alkylaminocarbonyl, di(C₁-C₄-alkyl)aminocarbonyl, aminocarbonyl, C₁-C₄-alkylcarbonyloxy or C₃-C₆cycloalkyl;
are phenyl, heterocyclyl, phenyl-C₁-C₆-alkyl, heterocyclyl-C₁-C₆-alkyl, phenoxy, heterocyclyloxy, where the phenyl and the heterocyclyl radical of the lastmentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R¹⁴ is C₁-C₆-alkyl, C₃-C₆-alkenyl, C₃-C₆-haloalkenyl, C₃-C₆-alkynyl, C₃-C₆-haloalkynyl, C₃-C₆-cycloalkyl, hydroxyl, C₁-C₆-alkoxy, C₃-C₆-alkenyloxy, C₃-C₆-alkynyloxy, amino, C₁-C₆-alkylamino, di(C₁-C₆-alkyl)amino or C₁-C₆-alkylcarbonylamino, where the abovementioned alkyl, cycloalkyl and alkoxy radicals may be partially or fully halogenated and/or may carry one to three radicals of the following group:
cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, di(C₁-C₄-alkyl)amino-C₁-C₄-alkoxycarbonyl, hydroxycarbonyl, C₁-C₄-alkylaminocarbonyl, di(C₁-C₄-alkyl)aminocarbonyl, aminocarbonyl, C₁-C₄-alkylcarbonyloxy or C₃-C₆-cycloalkyl;
is phenyl, heterocyclyl, phenyl-C₁-C₆-alkyl or heterocyclyl-C₁-C₆-alkyl, where the phenyl or heterocyclyl radical of the four lastmentioned substituents may be partially or fully halogenated and/or may carry one to three of the following radicals:
nitro, cyano, C₁-C₄-alkyl, C₁-C₄-haloalkyl, C₁-C₄-alkoxy or C₁-C₄-haloalkoxy;
R¹⁵ is C₁-C₆-alkyl, C₃-C₆-alkenyl, C₃-C₆-alkynyl or C₁-C₆-alkylcarbonyl;
and agriculturally useful salts thereof.

2. A process for preparing compounds of the formula I as claimed in claim 1 where R⁸ = halogen, which comprises reacting a pyrazolone derivative of the formula III, 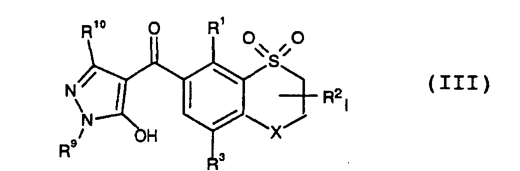 where the variables R¹ to R¹⁰, X, l are as defined in claim 1 with a halogenating agent.

3. A process for preparing compounds of the formula I as claimed in claim 1 where R⁸ = OR¹¹, OPOR¹²R¹³ or OPSR¹²R¹³, which comprises reacting a pyrazolone derivative of the formula III, 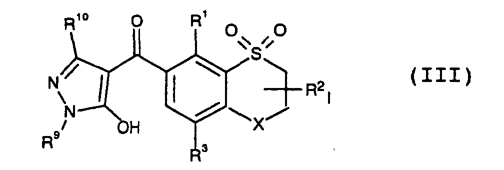 where the variables R¹ to R¹⁰, X, l are as defined in claim 1 with a compound of the formula IVα, IVβ or IVγ, 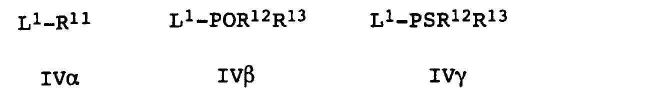 where the variables R¹¹ to R¹³ are as defined in claim 1 and L¹ is a nucleophilically replaceable leaving group.

4. A process for preparing compounds of the formula I as claimed in claim 1 where R⁸ = OR¹¹, SR¹¹, POR¹²R¹³, NR¹⁴R¹⁵, ONR¹⁵R¹⁵, N-bonded heterocyclyl or O-(N-bonded heterocyclyl), which comprises reacting a compound of the formula Iα (≡ I where R⁸ = halogen), 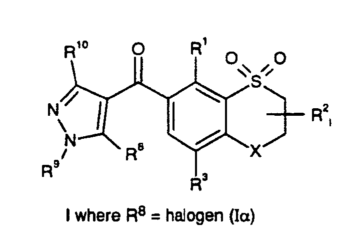 where the variables R¹ to R³, R⁹ and R¹⁰, X, l are as defined in claim 1 with a compound of the formula Vα, Vβ, Vγ, Vδ, Vε, Vη or Vϑ, 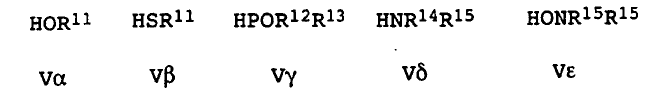 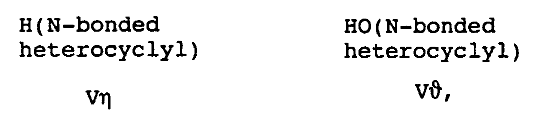 where the variables R¹¹ to R¹⁵ are as defined in claim 1, if appropriate in the presence of a base.

5. A process for preparing compounds of the formula I as claimed in claim 1 where R⁸ = SOR¹², SO₂R¹², which comprises reacting a compound of the formula Iβ (≡ I where R⁸ = SR¹²), 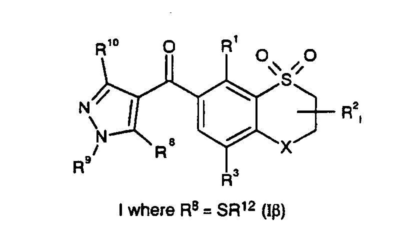 where the variables R¹ to R³, R⁹, R¹⁰, X, l are as defined in claim 1 with an oxidizing agent.

6. A process for preparing compounds of the formula I where R⁷ = IIa as claimed in claim 1, wherein a metallated pyrazole derivative of the formula VI where M is a metal and R⁸ to R¹⁰ are each as defined in claim 1 is reacted with a dioxothiochromancarboxylic acid derivative of the formula VII where R¹ to R³, X and l are each as defined in claim 1 and L² is a nucleophilically replaceable leaving group.

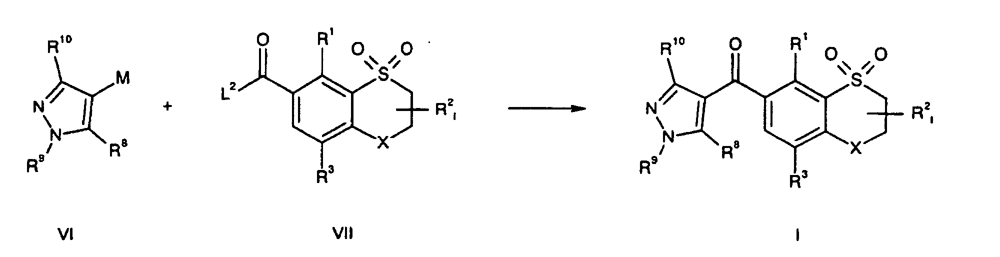

7. A composition, comprising a herbicidally effective amount of at least one pyrazolyldioxothiochromanoyl derivative of the formula I or an agriculturally useful salt of I as claimed in claim 1, and auxiliaries which are customary for the formulation of crop protection agents.

8. A process for preparing a composition as claimed in claim 7, which comprises mixing a herbicidally effective amount of at least one pyrazolyldioxothiochromanoyl derivative of the formula I or an agriculturally useful salt of I as claimed in claim 1 and auxiliaries which are customary for the formulation of crop protection agents.

9. A method for controlling undesirable vegetation, which comprises allowing a herbicidally effective amount of at least one pyrazolyldioxothiochromanoyl derivative of the formula I or an agriculturally useful salt of I as claimed in claim 1 to act on plants, their habitat and/or on seeds.

10. The use of pyrazolyldioxothiochromanoyl derivatives of the formula I or agriculturally useful salts thereof as claimed in claim 1 as herbicides.

## Revendications

1. Dérivés de pyrazolyldioxothiochromanoyle répondant à la formule I 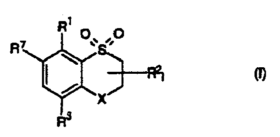 dans laquelle les variables possèdent les significations ci-après :
X représente un groupe S(=O)₂ ou un groupe CR⁴R⁵ ;
R¹ représente un atome d'hydrogène, un groupe nitro, un atome d'halogène, un groupe cyano, un groupe alkyle en C₁ - C₆, un groupe halogénalkyle en C₁ - C₆, un groupe alcoxy en C₁ - C₆, un groupe halogénalcoxy en C₁ - C₆, un groupe alkyl(en C₁ - C₆)-thio, un groupe halogénalkyl(en C₁ - C₆)thio, un groupe alkyl(en C₁ -C₆)sulfinyle, un groupe halogénalkyl(en C₁ - C₆)sulfinyle, un groupe alkyl(en C₁ - C₆)sulfonyle, un groupe halogénalkyl(en C₁-C₆)sulfonyle, un groupe aminosulfonyle, un groupe N-(alkyl en C₁ - C₆)aminosulfonyle, un groupe N,N-(dialkyl en C₁ - C₆)amino-sulfonyle, un groupe N-(alkyl(en C₁ - C₆)sulfonyl)-amino, un groupe N-(halogénalkyl(en C₁ - C₆)sulfonyl)-amino, un groupe N-(alkyl en C₁ - C₆)-N-(alkyl(en C₁ - C₆)sulfonyl)-amino ou un groupe N-(alkyl en C₁ - C₆)-N-(halogénalkyl(en C₁ - C₆)sulfonyl)-amino ;
R² représente un groupe alkyle en C₁ - C₆, un groupe hatogénalkyle en C₁ - C₆, un groupe alcoxy en C₁ - C₆, ou un groupe halogénalcoxy en C₁ - C₆;
R³ représente un atome d'hydrogène, un groupe alkyle en C₁ - C₆ ou un atome d'halogène;
R⁴, R⁵ représentent un atome d'hydrogène, un groupe nitro, un atome d'halogène, un groupe cyano, un groupe alkyle en C₁ - C₆, un groupe halogénalkyle en C₁ - C₆, un groupe alcoxy en C₁ - C₆, un groupe halogénalcoxy en C₁ - C₆, un groupe alkyl(en C₁ - C₆)-thio, un groupe halogénalkyl(en C₁ - C₆)thio, un groupe alkyl(en C₁ - C₆)sulfinyle, un groupe halogénalkyl(en C₁ - C₆)sulfinyle, un groupe alkyl(en C₁ - C₆)sulfonyle, un groupe halogénalkyl(en C₁-C₆)sulfonyle, un groupe N-alkyl(en C₁ - C₆)amino, un groupe N-halogénalkyl(en C₁ - C₆)amino, un groupe N,N-di(alkyl en C₁-C₆)amino, un groupe N-alcoxy(en C₁ - C₆)amino, un groupe N-(alcoxy en C₁ - C₆)-N-(alkyl en C₁ - C₆)amino, un groupe 1-tétrahydropyrrolyle, un groupe 1-pipéridinyle, un groupe 4-morpholinyle ou un groupe 1-hexahydropyrazinyle ;
ou
R⁴ et R⁵ forment ensemble une chaîne -O-(CH₂)ₘ-O-, une chaîne -O-(CH₂)ₘ-S-, une chaîne -S-(CH₂)ₘ-S- ou une chaîne -O-(CH₂)ₙ-, qui peut porter, à titre de substituants, de 1 à 3 radicaux choisis parmi le groupe ci-après comprenant :
un atome d'halogène, un groupe cyano, un groupe alkyle en C₁ - C₄, un groupe halogénalkyle en C₁ - C₄, ou un groupe alcoxy(en C₁ - C₄)-carbonyle ;
ou
R⁴ et R⁵ forment ensemble une chaîne -(CH₂)ₚ- qui peut être interrompue par un atome d'oxygène ou par un atome de soufre et/ou qui peut porter, à titre de substituants, de 1 à 4 radicaux choisis parmi le groupe ci-après comprenant
un atome d'halogène, un groupe cyano, un groupe alkyle en C₁ - C₄, un groupe halogénalkyle en C₁ - C₄ ou un groupe alcoxy(en C₁ - C₄) carbonyle ;
ou
R⁴ et R⁵ forment ensemble un groupe méthylidène qui peut porter, à titre de substituants de 1 à 2 radicaux choisis parmi le groupe ci-après comprenant:
un atome d'halogène, un groupe cyano, un groupe hydroxyle, un groupe formyle, un groupe alkyle en C₁ - C₆, un groupe halogénalkyle en C₁ - C₆, un groupe alcoxy en C₁ - C₆, un groupe halogénalcoxy en C₁ - C₆, un groupe alkyl(en C₁ - C₆)-thio, un groupe halogénalkyl(en C₁ - C₆)thio, un groupe alkyl(en C₁ - C₆)sulfinyle, un groupe halogénalkyl(en C₁ - C₆)sulfinyle, un groupe alkyl(en C₁ - C₆)sulfonyle ou un groupe halogénalkyl(en C₁ - C₆)sulfonyle;
l représente un nombre de 0 à 4 ;
m représente un nombre de 2 à 4 ;
n représente un nombre de 1 à 5 ;
p représente un nombre de 2 à 5;
R⁷ représente un composé lla 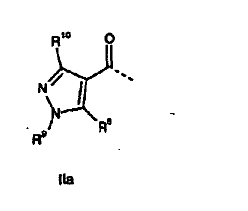
dans laquelle
R⁸ représente un atome d'halogène, un groupe OR¹¹, un groupe SR¹¹, un groupe SOR¹², un groupe SO₂R¹², un groupe POR¹²R¹³, un groupe OPOR¹²R¹³, un groupe OPSR¹²R¹³, un groupe NR¹⁴R¹⁵, un groupe ONR¹⁵R¹⁵, un groupe hétérocyclyle lié à un atome d'azote ou un groupe O-(hétérocyclyle lié à un atome d'azote), le radical hétérocyclyle des deux substituants mentionnés en dernier lieu pouvant être soumis à une halogénation partielle ou complète et/ou pouvant porter de 1 à 3 radicaux choisis parmi ceux repris ci-après
un groupe nitro, un groupe cyano, un groupe alkyle en C₁ - C₄, un groupe halogénalkyle en C₁ - C₄, un groupe alcoxy en C₁ - C₄ ou un groupe halogénalcoxy en C₁ - C₄;
R⁹ représente un atome d'hydrogène, un groupe alkyle en C₁ - C₆, un groupe halogénalkyle en C₁ - C₆, un groupe hydroxyle, un groupe alcoxy en C₁ - C₆, ou un groupe halogénalcoxy en C₁ - C₆ ;
R¹⁰ représente un atome d'hydrogène, un atome d'halogène, un groupe alkyle en C₁- C₆, un groupe halogénalkyle en C₁ - C₆, un groupe hydroxyle, un groupe alcoxy en C₁ - C₆, un groupe halogénalcoxy en C₁ - C₆, un groupe alkyl(en C₁ - C₆)thio, un groupe halogénalkyl(en C₁- C₆)thio;
R¹¹ représente un groupe alkyle en C₁ - C₆, un groupe alcényle en C₃ - C₆, un groupe halogénalcényle en C₃ - C₆, un groupe alcynyle en C₃ - C₆, un groupe halogénalcynyle en C₃ - C₆, un groupe cycloalkyle en C₃ - C₆, un groupe alkyl(en C₁ - C₂₀)carbonyle, un groupe alcényl(en C₂ - C₂₀)carbonyle, un groupe alcynyl(en C₂ - C₆)carbonyle, un groupe cycloalkyl(en C₃ - C₆)carbonyle, un groupe (2-norbornyl)méthylcarbonyle ; un groupe alcoxy(en C₁ - C₆)carbonyle, un groupe alcényl(en C₃ - C₆)oxycarbonyle, un groupe alcynyl(en C₃ - C₆)oxycarbonyle, un groupe alkyl(en C₁ - C₆)thiocarbonyle, un groupe alkyl(en C₁ - C₆)aminocarbonyle, un groupe alcényl(en C₃ - C₆)aminocarbonyle, un groupe alcynyl(en C₃ - C₆)aminocarbonyle, un groupe N,N-di(alkyl en C₁ - C₆)aminocarbonyle, un groupe N-(alcényl en C₃ - C₆)-N-(alkyl en C₁ - C₆)aminocarbonyle, un groupe N-(alcynyl en C₃ - C₆)-N- (alkyl en C₁ - C₆)aminocarbonyle, un groupe N-(alcoxy en C₁ - C₆)-N-(alkyl en C₁ - C₆)aminocarbonyle, un groupe N-(alcényl en C₃ - C₆)-N-(alcoxy en C₁ - C₆)aminocarbonyle, un groupe N- (alcynyl en C₃ - C₆)-N-(alcoxy en C₁ - C₆)aminocarbonyle, un groupe di(alkyl en C₁ - C₆)aminothiocarbonyle, un groupe alkyl(en C₁ - C₆)carbonylalkyle en C₁ - C₆, un groupe alcoxy(en C₁ - C₆)iminoalkyle en C₁ - C₆, un groupe N-(alkyl(en C₁ - C₆)amino)iminoalkyle en C₁ - C₆ ou un groupe N,N-di(alkyl(en C₁ - C₆)amino)iminoalkyle en C₁ - C₆, les radicaux alkyle, cycloalkyle et alcoxy mentionnés pouvant être soumis à une halogénation partielle ou complète et/ou pouvant porter de 1 à 3 groupes choisis parmi ceux repris ci-après :
un groupe cyano, un groupé alcoxy en C₁ - C₄, un groupe alkyl(en C₁ - C₄)thio, un groupe di(alkyl en C₁ - C₄)amino, un groupe alkyl(en C₁ - C₄)carbonyle, un groupe alcoxy(en C₁ - C₄)carbonyle, un groupe alcoxy(en C₁ - C₄)alcoxy(en C₁ - C₄)carbonyle, un groupe di(alkyl en C₁ - C₄)aminoalcoxy(en C₁ - C₄)carbonyle, un groupe hydroxycarbonyle, un groupe alkyl(en C₁ - C₄)aminocarbonyle, un groupe di(alkyl en C₁ - C₄)aminocarbonyle, un groupe aminocarbonyle, un groupe alkyl(en C₁ - C₄)carbonyloxy ou un groupe cycloalkyle en C₃ - C₆;
un groupe phényle, un groupe hétérocyclyle, un groupe phénylalkyle en C₁ - C₆, un groupe hétérocyclylalkyle en C₁ - C₆, un groupe phénylcarbonylalkyle en C₁ - C₆, un groupe hétérocyclylcarbonylalkyle en C₁ - C₆, un groupe phénylcarbonyie, un groupe hétérocyclylcarbonyle, un groupe phénoxycarbonyle, un groupe phényloxythiocarbonyle, un groupe hétérocyclyloxycarbonyle, un groupe hétérocyclyloxythiocarbonyle, un groupe phénylaminocarbonyle, un groupe N-(alkyl en C₁ - C₆)-N-(phényl)-aminocarbonyle, un groupe hétérocyclylaminocarbonyle, un groupe N-(alkyl en C₁ - C₆)-N-(hétérocyclyl)-aminocarbonyle, un groupe phénylalcényl(en C₂ - C₆)carbonyle ou un groupe hétérocyclylalcényl(en C₂ - C₆)carbonyle, le radical phényle et le radical hétérocyclyle des 18 derniers substituants pouvant être soumis à une halogénation partielle ou complète et/ou pouvant porter de 1 à 3 radicaux choisis parmi ceux indiqués ci-après :
un groupe nitro, un groupe cyano, un groupe alkyle en C₁ - C₄, un groupe halogénalkyle en C₁ - C₄, un groupe alcoxy en C₁ - C₄ , un groupe halogénalcoxy en C₁ - C₄, un groupe phénoxy, un groupe hétérocyclyle ou encore un groupe hétérocyclyle lié à un atome d'azote, les trois derniers substituants pouvant, à leur tour, être soumis à une halogénation partielle ou complète et/ou pouvant porter de 1 à 3 radicaux choisis parmi ceux indiqués ci-après: un groupe nitro, un groupe cyano, un groupe alkyle en C₁ - C₄, un groupe halogénalkyle en C₁ - C₄, un groupe alcoxy en C₁ - C₄ ou un groupe halogénalcoxy en C₁ - C₄ ;
R¹², R¹³ représentent un groupe alkyle en C₁ - C₆, un groupe alcényle en C₃ - C₆, un groupe halogénalcényle en C₃ - C₆, un groupe alcynyle en C₃ - C₆, un groupe halogénalcynyle en C₃ - C₆, un groupe cycloalkyle en C₃ - C₆, un groupe hydroxyle, un groupe alcoxy en C₁ - C₆, un groupe amino, un groupe alkyl(en C₁ - C₆)amino, un groupe halogénalkyl(en C₁ - C₆)amino, un groupe di(alkyl en C₁ - C₆)amino ou un groupe di(halogénalkyl en C₁ - C₆)amino, les radicaux alkyle, cycloalkyle et alcoxy mentionnés pouvant être soumis à une halogénation partielle ou complète et/ou pouvant porter de 1 à 3 groupes choisis parmi ceux repris ci-après :
un groupe cyano, un groupe alcoxy en C₁ - C₄, un groupe alkyl(en C₁ - C₄)thio, un groupe di(alkyl en C₁ - C₄)amino, un groupe alkyl(en C₁ - C₄)carbonyle, un groupe alcoxy(en C₁ - C₄)-carbonyle, un groupe alcoxy(en C₁ - C₄)alcoxy(en C₁ - C₄)-carbonyle, un groupe di(alkyl en C₁ - C₄)aminoalcoxy(en C₁ - C₄)carbonyle, un groupe hydroxycarbonyle, un groupe alkyl(en C₁ - C₄)aminocarbonyle, un groupe di(alkyl en C₁ - C₄)aminocarbonyle, un groupe aminocarbonyle, un groupe alkyl(en C₁ - C₄)carbonyloxy ou un groupe cycloalkyle en C₃ - C₆ ;
un groupe phényle, un groupe hétérocyclyle, un groupe phénylalkyle en C₁ - C₆, un groupe hétérocyclylalkyle en C₁ - C₆, un groupe phénoxy, un groupe hétérocyclyloxy, le radical phényle et le radical hétérocyclyle des substituants mentionnés en dernier lieu pouvant être soumis à une halogénation partielle ou complète et/ou pouvant porter de 1 à 3 radicaux choisis parmi ceux repris ci-après:
un groupe nitro, un groupe cyano, un groupe alkyle en C₁ - C₄, un groupe halogénalkyle en C₁ - C₄, un groupe alcoxy en C₁ - C₄ ou un groupe halogénalcoxy en C₁ - C₄ ;
R¹⁴ représente un groupe alkyle en C₁ - C₆, un groupe alcényle en C₃ - C₆, un groupe halogénalcényle en C₃ - C₆, un groupe alcynyle en C₃ - C₆, un groupe halogénalcynyle en C₃ - C₆, un groupe cycloalkyle en C₃- C₆, un groupe hydroxyle, un groupe alcoxy en C₁ - C₆, un groupe alcényl(en C₃ - C₆)oxy, un groupe alcynyl(en C₃- C₆)oxy, un groupe amino, un groupe alkyl(en C₁ - C₆)amino, un groupe di(alkyl en C₁ - C₆)amino ou un groupe alkyl(en C₁ - C₆)carbonylamino, les radicaux alkyle, cycloalkyle et alcoxy mentionnés pouvant être soumis à une halogénation partielle ou complète et/ou pouvant porter de 1 à 3 groupes choisis parmi ceux repris ci-après
un groupe cyano, un groupe alcoxy en C₁ - C₄, un groupe alkyl(en C₁ - C₄)thio, un groupe di(alkyl en C₁ - C₄)amino, un groupe alkyl(en C₁ - C₄)carbonyle, un groupe alcoxy(en C₁ - C₄)-carbonyle, un groupe alcoxy(en C₁ - C₄)alcoxy(en C₁ - C₄)-carbonyle, un groupe di(alkyl en C₁ - C₄)aminoalcoxy(en C₁ - C₄)carbonyle, un groupe hydroxycarbonyle, un groupe alkyl(en C₁ - C₄)aminocarbonyle, un groupe di(alkyl en C₁ - C₄)aminocarbonyle, un groupe aminocarbonyle, un groupe alkyl(en C₁-C₄)carbonyloxy ou un groupe cycloalkyle en C₃ - C₆;
un groupe phényle, groupe hétérocyclyle, un groupe phénylalkyle en C₁ - C₆ ou un groupe hétérocyclylalkyle en C₁ - C₆, le radical phényle ou le radical hétérocyclyle des quatre substituants mentionnés en dernier lieu pouvant être soumis à une halogénation partielle ou complète et/ou pouvant porter de 1 à 3 radicaux choisis parmi ceux repris ci-après :
un groupe nitro, un groupe cyano, un groupe alkyle en C₁ - C₄, un groupe halogénalkyle en C₁ - C₄, un groupe alcoxy en C₁ - C₄ ou un groupe halogénalcoxy en C₁ - C₄ ;
R¹⁵ représente un groupe alkyle en C₁ - C₆, un groupe alcényle en C₃ - C₆, un groupe alcynyle en C₃ - C₆ ou un groupe alkyl(en C₁ - C₆)carbonyle;
ainsi que leurs sels utiles en agriculture.

2. Procédé pour la préparation de composés répondant à la formule I selon la revendication 1, dans lesquels R⁸ représente un atome d'halogène **caractérisé en ce qu'**on fait réagir un dérivé de pyrazolone répondant à la formule III 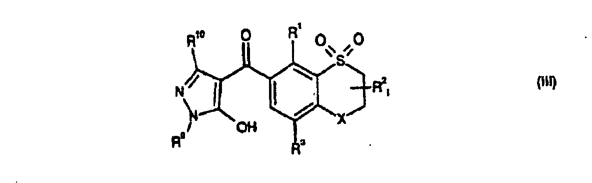 dans laquelle les variables R¹ à R¹⁰, X et I ont la signification mentionnée dans la revendication 1, avec un agent d'halogénation.

3. Procédé pour la préparation de composés répondant à la formule I selon la revendication 1, dans lesquels R⁸ représente un groupe OR¹¹, un groupe OPOR¹²R¹³ ou un groupe OPSR¹²R¹³, **caractérisé en ce qu'**on fait réagir un dérivé de pyrazolone répondant à la formule III 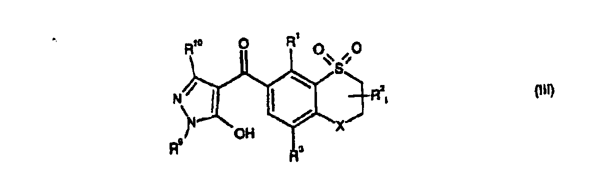 dans laquelle les variables R¹ à R¹⁰, X et I ont la signification mentionnée dans la revendication 1, avec un composé répondant aux formules IVα, IVβ ou IVγ, 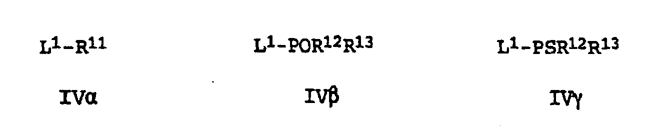 dans lesquelles les variables R¹¹ à R¹³ ont la signification mentionnée dans la revendication 1, et L¹ représente un groupe sortant apte à un déplacement nucléophile.

4. Procédé pour la préparation de composés répondant à la formule 1 selon la revendication 1, dans lesquels R⁸ représente un groupe OR¹¹, un groupe SR¹¹, un groupe POR¹²R¹³, un groupe NR¹⁴R¹⁵, un groupe 0NR^{1 5}R¹⁵, un groupe hétérocyclyle lié à un atome d'azote ou un groupe O-(hétérocyclyle lié à un atome d'azote), **caractérisé en ce qu'**on fait réagir un composé répondant à la formule lα (≡ l dans laquelle R⁸ représente un atome d'halogène), 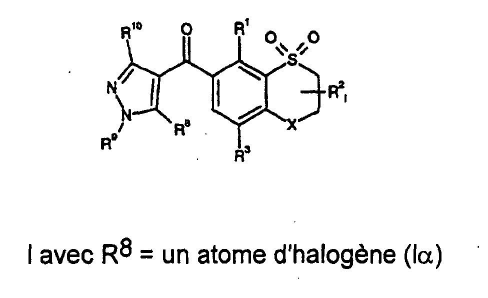 dans laquelle les variables R¹ à R³, R⁹ et R¹⁰, X et l ont la signification mentionnée dans la revendication 1, avec un composé répondant aux formules Vα, Vβ, Vγ, Vδ, Vε, Vη ou Vϑ, 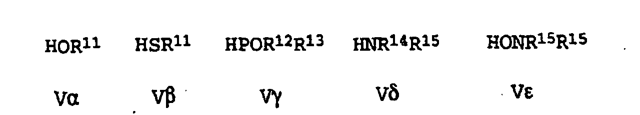 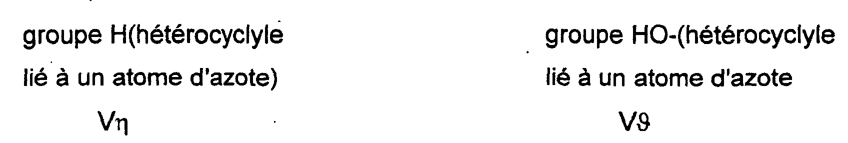 dans lesquelles les variables R¹¹ à R¹⁵ ont la signification mentionnée dans la revendication 1, le cas échéant en présence d'une base.

5. Procédé pour la préparation de composés répondant à la formule I selon la revendication 1, dans lesquels R⁸ représente un groupe SOR¹², un groupe SO₂R¹², **caractérisé en ce qu'**on fait réagir un composé répondant à la formule |β (≡ | dans laquelle R⁸ représente un groupe SR¹²), 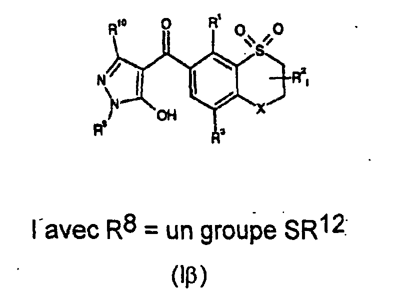 dans lesquelles les variables R¹ à R³, R⁹, R¹⁰, X et l ont la signification mentionnée dans la revendication 1, avec un agent d'oxydation.

6. Procédé pour la préparation de composés répondant à la formule I, dans lesquels R⁷ répond à la formule lla selon la revendication 1, **caractérisé en ce qu'**on fait réagir un dérivé de pyrazole métallisé répondant à la formule Vl, dans laquelle M représente un métal et R⁸ à R¹⁰ ont la signification mentionnée dans la revendication 1, avec un dérivé de l'acide dioxothiochromanecarboxylique répondant à la formule VII, dans laquelle R¹ à R³, X et I ont la signification mentionnée dans la revendication 1, et L² représente un groupe sortant apte à un déplacement nucléophile.

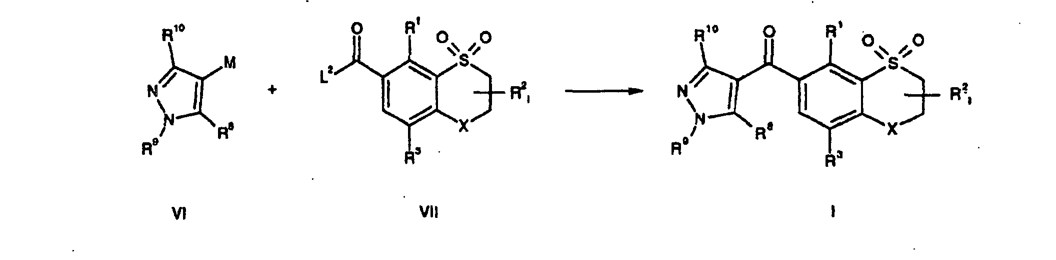

7. Agent contenant une quantité efficace du point de vue herbicide d'au moins un dérivé de pyrazolyldioxothiochromanoyle répondant à la formule I ou d'un sel utile en agriculture du dérivé répondant à la formule I selon la revendication 1, et des adjuvants habituels pour la formulation d'agents de protection des plantes.

8. Procédé pour la préparation d'agents selon la revendication 7, **caractérisé en ce qu'**on mélange une quantité efficace du point de vue herbicide d'au moins un dérivé de pyrazolyldioxothiochromanoyle répondant à la formule I ou d'un sel utile en agriculture du dérivé répondant à la formule I selon la revendication 1 et des adjuvants habituels pour la formulation d'agents de protection des plantes.

9. Procédé pour lutter pour la croissance non désirée de plantes, **caractérisé en ce qu'**on laisse agir une quantité efficace du point de vue herbicide d'au moins un dérivé de pyrazolyldioxothiochromanoyle répondant à la formule I ou d'un sel utile en agriculture du dérivé répondant à la formule I selon la revendication 1, sur des plantes, sur leur biotope et/ou sur des semences.

10. Utilisation de dérivés de pyrazolyldioxothiochromanoyle répondant à la formule 1 ou de leurs sels utiles en agriculture selon la revendication 1, à titre d'herbicides.
